# EUROPÄISCHE PATENTANMELDUNG

(11) **EP 3 889 196 A1**
(43) Veröffentlichungstag der Anmeldung: **06.10.2021**
(21) Anmeldenummer: 20167025.4
(22) Anmeldetag: 31.03.2020
(51) Int. Cl.: C08G 18/34, A61K 8/06, A61K 8/87, A61Q 17/00

(54) **BIOBASIERTE POLYURETHAN-DISPERSIONEN FÜR SONNENSCHUTZ-ANWENDUNGEN**

(71) Anmelder: Covestro Deutschland AG, 51373 Leverkusen (DE)
(72) Erfinder: VIALA, Sophie, 50937 Köln (DE); POTTIE, Laurence, 50823 Köln (DE); DOERR, Sebastian, 40593 Düsseldorf (DE); POCHOROVSKI, Igor, 51467 Bergisch Gladbach (DE)
(74) Vertreter: Levpat

(57) **Zusammenfassung**

Die vorliegende Erfindung betrifft Sonnenschutz-Zusammensetzungen, enthaltend spezielle Polyurethane bzw. wässrige Dispersionen, sowie die Verwendung der genannten Polyurethane zur Herstellung von Sonnenschutzprodukten.

## Beschreibung

Die vorliegende Erfindung betrifft Sonnenschutz-Zusammensetzungen, enthaltend spezielle Polyurethane bzw. wässrige Dispersionen, sowie die Verwendung der genannten Polyurethane zur Herstellung von Sonnenschutzprodukten.

Seit Jahren ist eine braune Haut ein Synonym für attraktive, gesunde, sportliche und erfolgreiche Mensch. Um diese zu erzielen, setzen die Menschen ihre Haut der Sonnenstrahlung aus. Jedoch wirken die Sonnenstrahlen schädigend auf die Haut, da sie je nach Wellenlänge unterschiedlich tief in die Haut eindringen. Die kurzwelligere Strahlung im Bereich UVB mit einer Wellenlänge von 280 bis 320 nm erreicht die oberste Hautschicht. Strahlen im UVB-Bereich verursachen Sonnenbrand und sind für ein erhöhtes Hautkrebsrisiko verantwortlich. Die längerwelligen UVA Strahlen mit einer Wellenlänge von 320 bis 400 nm dringen in tiefer gelegene Hautschichten ein. Sie führen zur Schädigung der Collagen- und Elastinfasern, die für die Struktur und die Festigkeit der Haut von wesentlicher Bedeutung sind. Dies führt darüber hinaus zu einer vorzeitigen Hautalterung, insbesondere zur Bildung von Falten und Fältchen, unregelmäßiges Relief der Haut etc. Zum Schutz der Haut gegenüber Sonnenstrahlungen wurden Lichtschutzfiltersubstanzen entwickelt, insbesondere UVA- und UVB-Filter, enthalten in Form von Positivlisten gemäß Anhang VI, Liste der in kosmetischen Mitteln zugelassenen UV-Filter) aus der Verordnung Nr. 1223/2009 des Europäischen Parlaments und des Rates vom 30. November 2009.

Die Sonnenschutzprodukte werden oft im Urlaub oder in der Freizeit am Strand oder während sportlichen Aktivitäten draußen verwendet, wo der Körper im Kontakt mit Wasser oder Schweiß ist. Daher besteht der Bedarf wasserfeste und/oder schweißfeste Sonnenschutzzusammensetzungen zu entwickeln. Die Herstellung solcher Produkte wird ermöglicht durch die Verwendung von ausgewählten Technologien wie zum Beispiel Wasser-in-Öl (W/O)-Emulsionen oder durch den Einsatz von hydrophoben Filmbildnern wie zum Beispiel alkylierten Polyvinylpyrrolidonen.

Die Anwendung von Polyurethanen in Sonnenschutzzusammensetzungen wurde im Stand der Technik schon beschrieben. Die DE 10223693 beschreibt die Anwendung von einem Polyurethanen, welche gebildet werden aus der Polyaddition von 3-Isocyanatomethyl-3,5,5-trimethylcyclohexyl-1-isocyanat und mehrwertigen Alkoholen, Glyceriden, Hydroxyestern, Silikonderivaten und/oder Aminen. Die EP 1214929 beschreibt die Anwendung eines filmbildenden, wasserlöslichen oder wasserdispergierbaren Polyurethans zur Verbesserung der Wasserfestigkeit einer kosmetischen oder dermatologischen Zubereitung, enthaltend mindestens einen UV-Filter. Die US 2003044364 beschreibt die Verwendung von Polyurethanen zur Verbesserung der Wasserfestigkeit von Sonnenschutzformulierungen.

Die Sonnenschutzprodukte des Stands der Technik haben jedoch eine Reihe von Nachteilen, beispielsweise sind wasserfeste W/O Emulsionen oft schwer zu verteilen und/oder hinterlassen nach der Anwendung ein unangenehmes wachsiges Hautgefühl. Des Weiteren kann der Einsatz von hydrophoben Filmbildnern das Hautgefühl von Sonnenschutzzusammensetzungen drastisch verändern. Die Zusammensetzungen bilden während der Verteilung auf der Haut oft Röllchen, so genannter "Balling Effect", und hinterlassen ein klebriges, fettiges und schmieriges Hautgefühl.

Der vorliegenden Erfindung liegt daher die Aufgabe zugrunde, eine Sonnenschutz-Zusammensetzung bereitzustellen, die Komponenten, insbesondere Polyurethane, enthält, die zu einem hohen Anteil aus erneuerbaren Quellen stammen. Des Weiteren war es Aufgabe der vorliegenden Erfindung, eine Sonnenschutz-Zusammensetzung bereitzustellen, die die Nachteile der aus dem Stand der Technik bekannten Zusammensetzungen nicht aufweist. Insbesondere soll erfindungsgemäß eine Sonnenschutz-Zusammensetzung bereitgestellt werden, die einen hohen Lichtschutzfaktor-Boosting Effekt und eine hohe Wasserfestigkeit aufweist. Gleichzeitig werden jedoch auch andere wichtige Eigenschaften wie leichtes Auftragen, Tragekomfort, nicht klebriges und schmieriges Hautgefühl sowie keine Röllchenbildung nicht vernachlässigt.

Überraschend wird die Aufgabe gelöst durch den Einsatz von speziellen Polyurethanen bzw. wässrigen Dispersionen davon, erhältlich durch Umsetzung eines oder mehrerer wasserunlöslicher, nicht wasserdispergierbarer, isocyanatfunktioneller Polyurethanprepolymere A mit einer oder mehreren aminofunktionellen Verbindungen B), dadurch gekennzeichnet, dass das Polyurethanprepolymere A) erhältlich ist durch Umsetzung von einem oder mehreren Polyesterpolyolen mit einer Glasübergangstemperatur T_{g} von mindestens -50 °C und einem oder mehreren Polyisocyanaten.

Die vorliegende Erfindung stellt somit eine Sonnenschutz-Zusammensetzung bereit, enthaltend mindestens ein Polyurethan, erhältlich durch Umsetzung eines oder mehrerer wasserunlöslicher, nicht wasserdispergierbarer, isocyanatfunktioneller Polyurethanprepolymere A mit einer oder mehreren aminofunktionellen Verbindungen B), dadurch gekennzeichnet, dass das Polyurethanprepolymere A) erhältlich ist durch Umsetzung von einem oder mehreren Polyesterpolyolen mit einer Glasübergangstemperatur T_{g} von mindestens -50 °C und einem oder mehreren Polyisocyanaten.

Bevorzugt stellt die vorliegende Erfindung eine erfindungsgemäße Sonnenschutz-Zusammensetzung bereit, enthaltend mindestens ein Polyurethan, erhältlich durch Umsetzung eines oder mehrerer isocyanatfunktioneller Polyurethanprepolymere A), welche im Wesentlichen weder ionische noch ionogene Gruppen aufweisen, mit einer oder mehreren aminofunktionellen Verbindungen B).

Im Rahmen der Erfindung bedeutet der Begriff "wasserunlösliches, nicht wasserdispergierbares Polyurethanprepolymer" insbesondere, dass die Wasserlöslichkeit des erfindungsgemäß verwendeten Prepolymers bei 23°C weniger als 10 g / Liter, bevorzugter weniger als 5 g / Liter beträgt und das Prepolymer bei 23° keine sedimentationsstabile Dispersion in Wasser, insbesondere entionisiertem Wasser, ergibt. Mit anderen Worten setzt sich das Prepolymer, beim Versuch es in Wasser zu dispergieren, ab.

Bevorzugt weist das erfindungsgemäß verwendete Polyurethanprepolymere A) endständige Isocyanatgruppen auf, d.h. die Isocyanatgruppen liegen an den Kettenenden des Prepolymers. Besonders bevorzugt weisen sämtliche Kettenenden eines Polymers Isocyanatgruppen auf.

Weiterhin weist das erfindungsgemäß verwendete Polyurethanprepolymere A) bevorzugt im Wesentlichen weder ionische noch ionogene, d.h. zur Bildung von ionischen Gruppen befähigte, Gruppen auf, d.h. der Gehalt an ionischen und ionogenen Gruppen liegt zweckmäßig unter 15 Milliequivalenten pro 100 g Polyurethanprepolymer A), bevorzugt unter 5 Milliequivalenten, besonders bevorzugt unter einem Milliequivalente und ganz besonders bevorzugt unter 0,1 Milliequivalenten pro 100 g Polyurethanprepolymer A).

Die aminofunktionellen Verbindungen B) werden bevorzugt aus primären und/oder sekundären Aminen und/oder Diaminen ausgewählt. Insbesondere umfassen die aminofunktionellen Verbindungen B) mindestens ein Diamin. Die aminofunktionellen Verbindungen B) werden bevorzugt aus aminofunktionellen Verbindungen B2), die ionische oder ionogene Gruppe aufweisen, und aminofunktionellen Verbindungen B1), die keine ionische oder ionogene Gruppe aufweisen, ausgewählt.

In einer besonders bevorzugten Ausführungsform der Erfindung umfassen die aminofunktionellen Verbindungen B) wenigstens eine aminofunktionelle Verbindung B2), die ionische und/oder ionogene Gruppen aufweist. Besonders bevorzugt wird als ionische und/oder ionogene Gruppe die Sulfonat- bzw. die Sulfonsäuregruppe, noch bevorzugter die Natriumsulfonatgruppe verwendet.

In einer weiteren bevorzugten Ausführungsform der Erfindung umfassen die aminofunktionellen Verbindungen B) sowohl aminofunktionelle Verbindungen B2), die ionische und/oder ionogene Gruppe aufweisen, als auch aminofunktionellen Verbindungen B1), die keine ionische oder ionogene Gruppe aufweisen.

Polyurethane im Sinne der Erfindung sind demnach polymere Verbindungen, die mindestens zwei, bevorzugt mindestens drei Urethan-Gruppen-haltige Wiederholungseinheiten aufweisen:

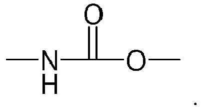

Erfindungsgemäß sind auch solche Polyurethane eingeschlossen, die herstellungsbedingt auch Harnstoff-Gruppen-haltige Wiederholungseinheiten: 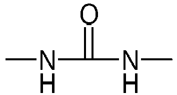 aufweisen, wie sie insbesondere bei der Umsetzung der isocyanatterminierten Prepolymere A) mit den aminofunktionellen Verbindungen B) gebildet werden.

Bei den erfindungsgemäßen Sonnenschutz-Zusammensetzungen kann es sich um wasserenthaltende, d.h. wässrige Zusammensetzungen handeln, in denen das Polyurethan dispergiert, d.h. im Wesentlichen nicht gelöst vorliegt. Wasser kann neben wahlweise vorhandenen anderen flüssigen Medien, wie beispielsweise Lösungsmittel, den Hauptbestandteil (> 50 Gew.-%) der Dispergiermedien, bezogen auf die Gesamtmenge der flüssigen Dispergiermedien in den erfindungsgemäßen Sonnenschutz-Zusammensetzungen, gegebenenfalls auch das alleinige flüssige Dispergiermedium sein.

Die erfindungsgemäßen Sonnenschutz-Zusammensetzungen weisen bevorzugt einen Gehalt an flüchtigen organischen Verbindungen (VOC) von weniger als 80 Gew.-%, bevorzugter von weniger als 55 Gew.-%, noch bevorzugter von weniger als 40 Gew.-% bezogen auf die dekorative kosmetische Zusammensetzung auf.

Die zur Herstellung der erfindungsgemäßen Sonnenschutz-Zusammensetzungen verwendeten wässrigen Polyurethandispersionen weisen bevorzugt einen Gehalt an flüchtigen organischen Verbindungen (VOC) von weniger als 10 Gew.-%, bevorzugter von weniger als 3 Gew.-%, noch bevorzugter von weniger als 1 Gew.-% bezogen auf die auf wässrige Polyurethandispersion auf.

Die Bestimmung des Gehalts an flüchtigen organischen Verbindungen (VOC) erfolgt im Rahmen der vorliegenden Erfindung insbesondere durch gaschromatographische Analyse.

Die erfindungsgemäß verwendeten nicht wasserlöslichen und nicht-wasserdispergierbaren, isocyanatfunktionellen Polyurethanprepolymere, weisen im Wesentlichen weder ionische noch ionogene Gruppen auf. Die Wasserunlöslichkeit bzw. fehlende Dispergierbakeit in Wasser bezieht sich auf entionisiertes Wasser ohne Zusatz von Tensiden. Im Rahmen der vorliegenden Erfindung bedeutet dies, dass der Anteil der ionischen und/oder ionogenen Gruppen, wie insbesondere anionischer Gruppen, wie Carboxylat oder Sulfonat, oder kationischer Gruppen weniger beträgt als 15 Milliequivalente pro 100 g Polyurethanprepolymer A), bevorzugt weniger als 5 Milliequivalente, besonders bevorzugt weniger als ein Milliequivalent und ganz besonders bevorzugt weniger als 0,1 Milliequivalente pro 100 g Polyurethanprepolymer A).

Im Falle saurer ionischer und/oder ionogener Gruppen beträgt die Säurezahl des Prepolymers zweckmäßig unter 30 mg KOH/g Prepolymer, bevorzugt unter 10 mg KOH/g Prepolymer. Die Säurezahl gibt die Masse Kaliumhydroxid in mg an, die zur Neutralisation von 1 g der zu untersuchenden Probe erforderlich ist, Messung nach DIN EN ISO 211. Die neutralisierten Säuren, also die entsprechenden Salze, weisen naturgemäß keine oder eine reduzierte Säurezahl auf. Hier ist erfindungsgemäß die Säurezahl der korrespondierenden freien Säure entscheidend.

Die zur Herstellung der Polyurethane verwendeten Prepolymere A) sind erhältlich durch die Umsetzung von einem oder mehreren Polyesterpolyolen mit einer Glasübergangstemperatur T_{g} von mindestens -50 °C und einem oder mehreren Polyisocyanaten. Die zur Herstellung der Prepolymere A) verwendeten ein oder mehrere Polyesterpolyole weisen weiter bevorzugt eine Glasübergangstemperatur T_{g} von -50 bis 0 °C, besonders bevorzugt -40 bis -10 °C, jeweils bestimmt durch DSC Messung gemäß DIN 65467 mit einer Heizrate von 20 K/min.

Die erfindungsgemäß eingesetzten Polyurethane weisen bevorzugt eine Glasübergangstemperatur Tg von mindestens -50 °C, besonders bevorzugt -50 bis 0 °C, insbesondere bevorzugt -30 bis 0 °C, ganz besonders bevorzugt -20 bis -10 °C, auf, jeweils bestimmt durch DSC Messung gemäß DIN 65467 mit einer Heizrate von 20 K/min.

Bevorzugt werden erfindungsgemäß ein oder mehrere Polyurethane eingesetzt, wobei mindestens 50 Gew.-% der Komponenten, die zum Aufbau des oder der Polyurethane eingesetzt werden, insbesondere das oder die Polyesterpolyole, weiter bevorzugt die zum Aufbau des oder der Polyesterpolyole eingesetzte Dicarbonsäuren und/oder Dihydroxyverbindungen, aus erneuerbaren Quellen stammen. Das resultierende Polymer, das mindestens zu 50 Gew.-% auf biobasierten Rohstoffen basiert, kann laut der Norm ISO 16128-1 als "naturally derived" ("Inhaltsstoffe natürlichen Ursprungs") qualifiziert werden.

Erfindungsgemäß weiter bevorzugt bedeutet der Begriff "aus erneuerbaren Quellen", dass für das entsprechende Material eine Quelle gewählt wird, die zu mindestens 90 Gew.-%, bevorzugt mindestens 95 Gew.-%, insbesondere bevorzugt mindestens 99 Gew.-% des Materials, welches "aus erneuerbaren Quellen" genannt wird, aus pflanzlichen oder Fermentations-Verfahren, in dem nur lebende Organismen und Pflanzen in dem Fermentationsverfahren teilnehmen.

Das oder die erfindungsgemäß eingesetzten Polyurethane sind bevorzugt zu mindestens 30 mol-%, besonders bevorzugt mindestens 40 mol-%, insbesondere bevorzugt mindestens 50 mol-%, aus erneuerbaren Quellen, d.h. biobasiert.

Die vorliegende Erfindung betrifft auch ein Polyurethan, erhältlich durch Umsetzung eines oder mehrerer wasserunlöslicher, nicht wasserdispergierbarer, isocyanatfunktioneller Polyurethanprepolymere A mit einer oder mehreren aminofunktionellen Verbindungen B), dadurch gekennzeichnet, dass das Polyurethanprepolymere A) erhältlich ist durch Umsetzung von einem oder mehreren Polyesterpolyolen mit einer Glasübergangstemperatur T_{g} von mindestens -50 °C und einem oder mehreren Polyisocyanaten. Zu den erfindungsgemäßen Polyurethanen gilt das bezüglich der erfindungsgemäßen Zusammensetzung entsprechend.

Die in den erfindungsgemäßen Sonnenschutz-Zusammensetzungen enthaltenen Polyurethane enthalten via das Prepolymer A) ein oder mehrere der oben genannten Polyesterpole biobasierter Herkunft mit der beschriebenen speziellen Glasübergangstemperatur. Zusätzlich können die erfindungsgemäß vorliegenden Prepolymere mindestens eine Sequenz ausgewählt wird aus der Gruppe bestehend aus Polyether-, Polycarbonat-, Polyether-Polycarbonat- und weiteren PolyesterSequenzen. Dies kann erfindungsgemäß bedeuten, dass die Polyurethane Ethergruppen- und/oder Carbonatgruppen-haltige und Estergruppen-Wiederholungseinheiten enthalten. Die erfindungsgemäß vorliegenden Polyurethane können beispielsweise ausschließlich Polyestersequenzen basierend auf den oben beschrieben Polyesterpolyolen mit einer Glasübergangstemperatur T_{g} von mindestens -50 °C enthalten. Sie können aber auch zusätzlich Polyether- als auch Polycarbonat-Sequenzen aufweisen, wie sie beispielsweise bei der Herstellung von Polycarbonat-Polyolen unter Verwendung von Polyetherdiolen gebildet werden. Zusätzlich können sie Polyether-Polycarbonat-Sequenzen aufweisen, welche aus der Verwendung von Polyether-Polycarbonat-Polyolen, wie weiter unten näher beschrieben, hervorgehen.

Erfindungsgemäß bevorzugte Polyurethane werden unter Verwendung von polymeren Polyesterpolyolen mit einer Glasübergangstemperatur T_{g} von mindestens -50 °C erhalten, welche zahlenmittlere Molekulargewichte von bevorzugt etwa 400 bis etwa 6000 g/mol, hier und bei den folgenden Molekulargewichtangaben bestimmt durch Gelpermeationschromatographie gegenüber Polystyrol-Standard in Tetrahydofuran bei 23 °C, aufweisen. Ihre Verwendung bei der Herstellung der Polyurethane bzw. Polyurethanprepolymere führt durch Umsetzung mit Polyisocyanaten zur Bildung entsprechender Polyestersequenzen in den Polyurethanen mit entsprechendem Molgewicht dieser Sequenzen. Besonders bevorzugt sind erfindungsgemäß Polyurethane, die aus polymeren Polyesterpolyolen mit einer Glasübergangstemperatur T_{g} von mindestens -50 °C mit linearem Aufbau und gegebenenfalls zusätzlich Polyether-Diolen und/oder polymeren Polycarbonat-Diolen und/oder Polyether-Polycarbonat-Polyolen oder weiteren Polyesterpolyolen erhalten werden.

Die erfindungsgemäßen Polyurethane sind bevorzugt im Wesentlichen lineare Moleküle, können jedoch auch verzweigt sein, was weniger bevorzugt ist.

Das zahlenmittlere Molekulargewicht der erfindungsgemäß bevorzugt verwendeten Polyurethane beträgt beispielsweise etwa von 1000 bis 200000 g/mol, bevorzugt von 5000 bis 150000 g/mol.

Die in den erfindungsgemäßen Sonnenschutz-Zusammensetzungen enthaltenen Polyurethane werden den genannten Zusammensetzungen insbesondere als wässrige Dispersionen zugesetzt.

Bevorzugte erfindungsgemäß einzusetzenden Polyurethane bzw. Polyurethan-Dispersionen sind erhältlich, in dem
A) isocyanatfunktionelle Prepolymere aus
   A1) organischen Polyisocyanaten,
   A2) polymeren Polyesterpolyolen mit einer Glasübergangstemperatur T_{g} von mindestens -50 °C, bevorzugt mit zahlenmittleren Molekulargewichten von 400 bis 8000 g/mol, hier und bei den folgenden Molekulargewichtangaben bestimmt durch Gelpermeationschromatographie gegenüber Polystyrol-Standard in Tetrahydofuran bei 23°C, bevorzugter 400 bis 6000 g/mol und besonders bevorzugt von 600 bis 3000 g/mol, und OH-Funktionalitäten von bevorzugt 1,5 bis 6, bevorzugter 1,8 bis 3, besonders bevorzugt von 1,9 bis 2,1,
   A3) gegebenenfalls hydroxyfunktionellen Verbindungen mit Molekulargewichten von bevorzugt 62 bis 399 g/mol, und
   A4) gegebenenfalls nichtionischen Hydrophilierungsmitteln, hergestellt werden, und
B) deren freie NCO-Gruppen dann ganz oder teilweise
   mit einer oder mehreren aminofunktionellen Verbindungen B), wie primären und/oder sekundären Aminen und/oder Diaminen, umsetzt.

Die erfindungsgemäß verwendeten Polyurethane werden bevorzugt vor, während oder nach Schritt B) in Wasser dispergiert.

Besondere bevorzugt erfolgt im Schritt B) die Umsetzung mit einem Diamin oder mehreren Diaminen unter Kettenverlängerung. Dabei können zusätzlich monofunktionelle Amine als Kettenabbrecher zur Molekulargewichtssteuerung zugesetzt werden.

Als Komponente B) können insbesondere Amine verwendet werden, die keine ionischen bzw. ionogenen, wie anionisch hydrophilierende Gruppen aufweisen, im folgenden Komponente B1), und es können Amine verwendet werden, die ionische bzw. ionogene, wie insbesondere anionisch hydrophilierenden Gruppen aufweisen, im folgenden Komponente B2).

Bevorzugt wird im Schritt B) der Umsetzung des Prepolymers eine Mischung aus Komponente B1) und Komponente B2) zur Umsetzung gebracht. Durch die Verwendung der Komponente B1) kann eine hohe Molmasse aufgebaut werden, ohne dass die Viskosität des zuvor hergestellten isocyanatfunktionellen Präpolymers in einem Maße ansteigt, welches einer Verarbeitung entgegenstehen würde. Durch die Verwendung der Kombination der Komponenten B1) und B2) lässt sich eine optimale Balance zwischen Hydrophilie und Kettenlänge und damit ein angenehmes Hautgefühl einstellen.

Die erfindungsgemäß verwendeten Polyurethane weisen bevorzugt anionische Gruppen, bevorzugt Sulfonatgruppen auf. Diese anionischen Gruppen werden in die erfindungsgemäß verwendeten Polyurethanen über die in Schritt B) umgesetzte Aminkomponente B2) eingeführt. Die erfindungsgemäß verwendeten Polyurethane weisen gegebenenfalls zusätzlich nicht-ionische Komponenten zu Hydrophilierung auf. Besonders bevorzugt sind in den erfindungsgemäß verwendeten Polyurethanen zur Hydrophilierung ausschließlich Sulfonatgruppen enthalten, welche über entsprechende Diamine als Komponente B2) in das Polyurethan eingeführt werden.

Um eine gute Sedimentationsstabilität zu erreichen, liegt die zahlenmittlere Teilchengröße der speziellen Polyurethan-Dispersionen bevorzugt bei weniger als 750 nm, besonders bevorzugt bei weniger als 500 nm, bestimmt mittels Laserkorrelations-Spektroskopie nach Verdünnung mit entionisiertem Wasser, verwendetes Gerät Malvern Zetasizer 1000, Malver Inst. Limited.

Der Feststoffgehalt der Polyurethan-Dispersionen, welche zur Herstellung der erfindungsgemäßen Sonnenschutz-Zusammensetzung bevorzugt verwendet wird, beträgt im Allgemeinen 10 bis 70 Gew.-%, bevorzugt 30 bis 65 Gew.-%, besonders bevorzugt 30 bis 50 Gew.-%. Die Feststoffgehalte werden erfindungsgemäß durch Erhitzen einer ausgewogenen Probe auf 125 °C bis zur Gewichtskonstanz ermittelt. Bei Gewichtskonstanz wird durch erneutes Auswiegen der Probe der Festkörpergehalt berechnet.

Bevorzugt weisen diese Polyurethan-Dispersionen weniger als 5 Gew.-%, besonders bevorzugt weniger als 0,2 Gew.-%, bezogen auf die Masse der Dispersionen, an ungebundenen organischen Aminen auf. Der Gehalt in den dekorativen kosmetischen Zusammensetzungen ist entsprechend noch geringer.

Geeignete Polyisocyanate der Komponente A1) sind insbesondere die dem Fachmann an sich bekannten aliphatischen, aromatischen oder cycloaliphatischen Polyisocyanate einer NCO-Funktionalität von größer oder gleich 2.

Beispiele solcher geeigneten Polyisocyanate sind 1,4-Butylendiisocyanat, 1,6-Hexamethylendiisocyanat (HDI), Isophorondiisocyanat (IPDI), 2,2,4- und/oder 2,4,4-Trimethylhexamethylendiisocyanat, die isomeren Bis-(4,4'-isocyanatocyclohexyl)methane oder deren Mischungen beliebigen Isomerengehalts, 1,4-Cyclohexylendiisocyanat, 4-Isocyanatomethyl-1,8-octandiisocyanat (Nonantriisocyanat), 1,4-Phenylendiisocyanat, 2,4- und/oder 2,6-Toluylendiisocyanat, 1,5-Naphthylendiisocyanat, 2,2'-und/oder 2,4'- und/oder 4,4'-Diphenylmethandiisocyanat, 1,3- und/oder 1,4-Bis-(2-isocyanato-prop-2-yl)-benzol (TMXDI), 1,3-Bis(isocyanato-methyl)benzol (XDI) sowie Alkyl-2,6-diisocyanatohexanoate (Lysindiisocyanate) mit C1-C8-Alkylgruppen.

Neben den vorstehend genannten Polyisocyanaten können auch modifizierte Diisocyanate die eine Funktionalität ≥ 2 aufweisen, mit Uretdion-, Isocyanurat-, Urethan-, Allophanat-, Biuret-, Iminooxadiazindion- oder Oxadiazintrionstruktur sowie Mischungen aus diesen anteilig eingesetzt werden.

Bevorzugt handelt es sich um Polyisocyanate oder Polyisocyanatgemische der vorstehend genannten Art mit ausschließlich aliphatisch oder cycloaliphatisch gebundenen Isocyanatgruppen oder Mischungen aus diesen und einer mittleren NCO-Funktionalität der Mischung von 2 bis 4, bevorzugt 2 bis 2,6 und besonders bevorzugt 2 bis 2,4, ganz besonders bevorzugt 2.

Besonders bevorzugt werden in A1) Hexamethylendiisocyanat, Isophorondiisocyanat oder die isomeren Bis-(4,4'-isocyanatocyclohexyl)methane sowie Mischungen der vorgenannten Diisocyanate eingesetzt.

In A2) werden polymere Polyesterpolyole mit einer Glasübergangstemperatur T_{g} von mindestens - 50 °C und mit einem zahlenmittleren Molekulargewicht Mₙ von bevorzugt 400 bis 8000 g/mol, bevorzugter von 400 bis 6000 g/mol und besonders bevorzugt von 600 bis 3000 g/mol eingesetzt. Diese weisen bevorzugt eine OH-Funktionalität von 1,5 bis 6, besonders bevorzugt von 1,8 bis 3, ganz besonders bevorzugt von 1,9 bis 2,1 auf.

Der Ausdruck "polymere" Polyesterpolyole bedeutet hier insbesondere, dass die genannten Polyole mindestens zwei, bevorzugter mindestens drei miteinander verbundene Wiederholungseinheiten aufweisen.

Diese erfindungsgemäßen Polyesterpolyole mit einer Glasübergangstemperatur T_{g} von mindestens -50 °C können in A2) einzeln oder in beliebigen Mischungen untereinander eingesetzt werden. Weitere Polyole, die gegebenenfalls zusätzlich vorliegen können, sind die dem Fachmann an sich bekannten Polyesterpolyole, Polyacrylatpolyole, Polyurethanpolyole, Polycarbonatpolyole, Polyetherpolyole, Polyesterpolyacrylatpolyole, Polyurethanpolyacrylatpolyole, Polyurethanpolyesterpolyole, Polyurethanpolyetherpolyole, Polyurethanpolycarbonatpolyole und Polyesterpolycarbonatpolyole.

Die erfindungsgemäß verwendeten Polyesterpolyole sind die an sich bekannten Polykondensate aus Di- sowie gegebenenfalls Tri- und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Beispiele für geeignete Diole sind Ethylenglykol, Butylenglykol, Diethylenglykol, Triethylenglykol, Polyalkylenglykole wie Polyethylenglykol, weiterhin 1,2-Propandiol, 1,3-Propandiol, Butandiol(1,3), Butandiol(1,4), Hexandiol(1,6) und Isomere, Neopentylglykol oder Hydroxypivalinsäureneopentylglykolester, wobei Hexandiol(1,6) und Isomere, Butandiol(1,4), Neopentylglykol und Hydroxypivalinsäureneopentylglykolester bevorzugt sind. Daneben können auch Polyole wie Trimethylolpropan, Glycerin, Erythrit, Pentaerythrit, Trimetylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Bernsteinsäure, Korksäure, 2-Methylbernsteinsäure, 3,3-Diethylglutarsäure und/oder 2,2-Dimethylbernsteinsäure eingesetzt werden. Als Säurequelle können auch die entsprechenden Anhydride verwendet werden.

Polyole, die mehr als zwei OH-Gruppen aufweisen, sind weiter oben beschrieben. Anstelle von oder zusätzlich dazu können auch Polyole wie Trimethylolpropan, Glycerin, Erythrit, Pentaerythrit, Trimethylolbenzol oder Trishydroxyethylisocyanurat eingesetzt werden. Sofern die mittlere Funktionalität des zu veresternden Polyols > als 2 ist, können zusätzlich auch Monocarbonsäuren, wie Benzoesäure und Hexancarbonsäure mit verwendet werden.

Bevorzugte Säuren sind aliphatische oder aromatische, besonders bevorzugt aliphatische, Säuren der vorstehend genannten Art. Besonders bevorzugt sind Bernsteinsäure, Adipinsäure, Isophthalsäure und Phthalsäure.

Bernsteinsäure, die bevorzugt für die Herstellung der erfindungsgemäß eingesetzten Polyesterpolyole verwendet wird, wird bevorzugt aus erneuerbaren Quellen gewonnen. Die Herstellung von Bernsteinsäure erfolgt dabei beispielsweise durch Fermentation von Stärke oder Biomasse, wie beispielsweise in DE 10 2008 051727 A1 und DE 10 2007 019184 beschrieben. Bevorzugt stammen erfindungsgemäß mindestens 50 Gew.-% der eingesetzten Bernsteinsäure aus erneuerbaren Quellen. Des Weiteren oder zusätzlich kann auch wenigstens ein Teil der in dem erfindungsgemäßen Polyesterpolyol eingesetzten Dihydroxyverbindungen aus erneuerbaren Quellen stammen, und somit den Anteil der Polyurethan-Komponenten, die aus erneuerbaren Quellen stammen, erhöhen. Bevorzugt liegt in dem erfindungsgemäß eingesetzten Polyesterpolyol wenigstens eine Dihydroxyverbindung ausgewählt aus 1,4-Butandiol, 1,3-Propandiol, iso-Propandiol, 1,6-Hexandiol, Ethylenglykol, welche weiter bevorzugt aus erneuerbaren Quellen stammen, und Mischungen davon, vor,

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe. Bevorzugt ist Caprolacton.

Erfindungsgemäß besonders bevorzugt sind als Komponente A2) zur Herstellung der Polyurethane Polyesterpolyole mit einer Glasübergangstemperatur T_{g} von mindestens -50 °C mit einem zahlenmittleren Molekulargewicht von 600 bis 3000 g/mol, insbesondere aliphatische Polyesterpolyole auf Basis aliphatischer Carbonsäuren und aliphatischer Polyole, insbesondere auf Basis von Bernsteinsäure oder Adipinsäure und aliphatischen Alkoholen, wie Butandiol(1,4), Hexandiol(1,6) und/oder Neopentylglykol.

Neben dem oben als Komponente A2) beschriebenen Polyesterpolyol können in dem erfindungsgemäß eingesetzten Polyurethan gegebenenfalls weitere Polyole vorliegen, beispielsweise hydroxylgruppenaufweisende Polycarbonate, bevorzugt Polycarbonatdiole, mit zahlenmittleren Molekulargewichten Mₙ von bevorzugt 400 bis 8000 g/mol, bevorzugt 600 bis 3000 g/mol eingesetzt werden. Diese sind durch Reaktion von Kohlensäurederivaten, wie Diphenylcarbonat, Dimethylcarbonat oder Phosgen, mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propan-diol, 2,2,4-Trimethylpentandiol-1,3, Dipropylenglykol, Polypropylenglykole, Dibutylenglykol, Polybutylenglykole, Bisphenol A und lactonmodifizierte Diole der vorstehend genannten Art.

Bevorzugt enthält die Diolkomponente 40 bis 100 Gew.-% Hexandiol, bevorzugt sind 1,6-Hexandiol und/oder Hexandiolderivate. Solche Hexandiolderivate basieren auf Hexandiol und weisen neben endständigen OH-Gruppen Ester- oder Ethergruppen auf. Solche Derivate sind durch Reaktion von Hexandiol mit überschüssigem Caprolacton oder durch Veretherung von Hexandiol mit sich selbst zum Di- oder Trihexylenglykol erhältlich.

Statt oder zusätzlich zu reinen Polycarbonatdiolen können auch Polyether-Polycarbonatdiole eingesetzt werden.

Hydroxylgruppen aufweisende Polycarbonate sind bevorzugt linear gebaut.

Ebenfalls können zusätzlich zu den erfindungsgemäßen Polyesterpolyolen Polyetherpolyole eingesetzt werden.

Besonders geeignet sind beispielsweise die in der Polyurethanchemie an sich bekannten Polytetramethylenglykolpolyether wie sie durch Polymerisation von Tetrahydrofuran mittels kationischer Ringöffnung erhältlich sind.

Ebenfalls geeignete Polyetherpolyole sind die an sich bekannten Additionsprodukte von Styroloxid, Ethylenoxid, Propylenoxid, Butylenoxid und/oder Epichlorhydrin an di- oder polyfunktionelle Startermoleküle. So sind insbesondere Polyalkylenglykole, wie Polyethylen-, Polypropylen- und/oder Polybutylenglykole anwendbar, insbesondere mit den oben genannten bevorzugten Molekulargewichten.

Als geeignete Startermoleküle können alle dem Stand der Technik nach bekannten Verbindungen eingesetzt werden, wie zum Beispiel Wasser, Butyldiglykol, Glycerin, Diethylenglykol, Trimethyolpropan, Propylenglykol, Sorbit, Ethylendiamin, Triethanolamin, 1,4-Butandiol.

Besonders bevorzugte Polyole, die zusätzlich zu Komponente A2) vorliegen können, sind Polytetramethylenglykolpolyether und Polycarbonat-Polyole bzw. deren Mischungen und besonders bevorzugt sind Polytetramethylenglykolpolyether.

In einer bevorzugten Ausführungsform der vorliegenden Erfindung können zusätzlich zu dem erfindungsgemäßen Polyesterpolyol (Komponente A2) die folgenden Polyolmischungen eingesetzt werden: Mischungen, enthaltend wenigstens ein Polyether-Polyol und wenigstens ein Polycarbonat-Polyol, Mischungen, enthaltend mehr als ein Polyether-Polyol, bzw. ein Gemisch mehrerer Polyether-Polyole mit unterschiedlichen Molekulargewichten, wobei es sich insbesondere um Poly(tetramethylenglykol)polyetherpolyole (wie (HO-(CH₂-CH₂-CH₂-CH₂-O)ₓ-H) handelt, Mischungen, enthaltend mehr als ein Polyether-Polyol, und wenigstens ein Polycarbonat-Polyol, sowie die oben genannten Polyesterpole, wobei die Polyolkomponente definitionsgemäß im Wesentlichen weder ionische noch ionogene Gruppen aufweist.

Als Komponente A3) können wahlweise Polyole, insbesondere nicht-polymere Polyole, des bevorzugt genannten Molekulargewichtsbereichs von 62 bis 399 mol/g mit bis zu 20 Kohlenstoffatomen, wie Ethylenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A (2,2-Bis(4-hydroxycyclohexyl)propan), Trimethylolpropan, Trimethylolethan, Glycerin, Pentaerythrit sowie deren beliebige Mischungen untereinander, insbesondere Neopentylglykol, eingesetzt werden.

Geeignet sind auch Esterdiole des genannten Molekulargewichtsbereichs wie α-Hydroxybutyl-ε-hydroxy-capronsäureester, ω-Hydroxyhexyl-γ-hydroxybuttersäureester, Adipinsäure-(β-hydroxyethyl)ester oder Terephthalsäurebis(β-hydroxyethyl)-ester.

Ferner können als Komponente A3) auch monofunktionelle isocyanatreaktive Hydroxylgruppenhaltige Verbindungen eingesetzt werden. Beispiele solcher monofunktionellen Verbindungen sind Ethanol, n-Butanol, Ethylenglykolmonobutylether, Diethylenglykolmonomethylether, Diethylenglykolmonobutylether, Propylenglykolmonomethylether, Dipropylenglykolmonomethylether, Tripropylenglykolmonomethylether, Dipropylenglykolmono-propylether, Propylenglykolmonobutylether, Dipropylenglykolmonobutylether, Tripropylenglykolmonobutylether, 2-Ethylhexanol, 1-Octanol, 1-Dodecanol, 1-Hexadecanol.

In einer bevorzugten Ausführungsform der Erfindung enthält dass erfindungsgemäß verwendete Polyurethan weniger als etwa 10 Gew.-% der Komponente A3), bevorzugt weniger als 5 Gew.-% der Komponente A3), jeweils bezogen auf die Gesamtmasse des Polyurethans, noch bevorzugter wird Komponente A3) zur Herstellung des Polyurethans nicht verwendet.

Als Komponente A4) werden zur Herstellung der erfindungsgemäß verwendeten Polyurethane gegebenenfalls ein oder mehrere insbesondere isocyanatreaktive nichtionische Hydrophilierungsmittel verwendet. Die als Komponente A4) verwendeten Hydrophilierungsmittel sind insbesondere von den Komponenten A2) und A3) verschieden.

Geeignete nichtionisch hydrophilierende Verbindungen als Komponente A4) sind z.B. Polyoxyalkylenether, welche über isocyanatreaktive Gruppen, wie Hydroxy-, Amino- oder Thiolgruppen verfügen. Bevorzugt sind monohydroxyfunktionelle, im statistischen Mittel 5 bis 70, bevorzugt 7 bis 55 Ethylenoxideinheiten pro Molekül aufweisenden Polyalkylenoxidpolyetheralkohole, wie sie in an sich bekannter Weise durch Alkoxylierung geeigneter Startermoleküle zugänglich sind (z.B. in Ullmanns Encyclopädie der technischen Chemie, 4. Auflage, Band 19, Verlag Chemie, Weinheim S. 31-38). Diese sind entweder reine Polyethylenoxidether oder gemischte Polyalkylenoxidether, wobei sie mindestens 30 mol-%, bevorzugt mindestens 40 mol-% bezogen auf alle enthaltenen Alkylenoxideinheiten an Ethylenoxideinheiten enthalten.

Besonders bevorzugte nichtionische Verbindungen sind monofunktionelle gemischte Polyalkylenoxidpolyether, die 40 bis 100 mol-% Ethylenoxid- und 0 bis 60 mol-% Propylenoxideinheiten aufweisen.

Geeignete Startermoleküle für solche nichtionischen Hydrophilierungsmittel sind insbesondere gesättigte Monoalkohole wie Methanol, Ethanol, n-Propanol, Isopropanol, n-Butanol, Isobutanol, sec-Butanol, die isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykol-monoalkylether, wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol, sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole der vorstehend genannten Art. Besonders bevorzugt werden Diethylenglykolmonobutylether oder n-Butanol als Startermoleküle verwendet.

Für die Alkoxylierungsreaktion geeignete Alkylenoxide sind insbesondere Ethylenoxid und Propylenoxid, die in beliebiger Reihenfolge oder auch im Gemisch bei der Alkoxylierungsreaktion eingesetzt werden können.

Die Komponente B) wird bevorzugt aus primären oder sekundären Amin und/oder Diaminen ausgewählt. Sie umfasst insbesondere Diamine.

Als Komponente B) können insbesondere Amine verwendet werden, die keine ionischen bzw. ionogenen, wie anionisch hydrophilierenden Gruppen aufweisen (im folgenden Komponente B1)), und es können Amine verwendet werden, die ionische bzw. ionogene, wie insbesondere anionisch hydrophilierenden Gruppen aufweisen (im folgenden Komponente B2)). Bevorzugt wird im Schritt B) der Umsetzung des Prepolymers eine Mischung aus der Komponente B1) und der Komponente B2) zur Umsetzung gebracht.

Beispielsweise können als Komponente B1) organische Di- oder Polyamine wie beispielsweise 1,2-Ethylendiamin, 1,2- und 1,3-Diaminopropan, 1,4-Diaminobutan, 1,6-Diaminohexan, Isophorondiamin, Isomerengemisch von 2,2,4- und 2,4,4-Trimethylhexamethylendiamin, 2-Methylpentamethylendiamin, Diethylentriamin, 4,4-Diaminodicyclohexylmethan, Hydrazinhydrat, und/oder Dimethylethylendiamin eingesetzt werden.

Darüber hinaus können als Komponente B1) auch Verbindungen, die neben einer primären Aminogruppe auch sekundäre Aminogruppen oder neben einer Aminogruppe (primär oder sekundär) auch OH-Gruppen aufweisen, eingesetzt werden. Beispiele hierfür sind primäre/sekundäre Amine, wie Diethanolamin, 3-Amino-1-methylaminopropan, 3-Amino-1-ethylaminopropan, 3-Amino-1-cyclohexylaminopropan, 3-Amino-1- methylaminobutan, Alkanolamine wie N-Aminoethylethanolamin, Ethanolamin, 3-Aminopropanol, Neopentanolamin.

Ferner können als Komponente B1) auch monofunktionelle isocyanatreaktive Aminverbindungen eingesetzt werden, wie beispielsweise Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, bzw. geeignete substituierte Derivate davon, Amidamine aus diprimären Aminen und Monocarbonsäuren, Monoketim von diprimären Aminen, primär/tertiäre Amine, wie N,N-Dimethylaminopropylamin.

Bevorzugt werden als Komponente B1) 1,2-Ethylendiamin, Bis(4-aminocyclohexyl)methan, 1,4-Diaminobutan, Isophorondiamin, Ethanolamin, Diethanolamin und Diethylentriamin eingesetzt.

Besonders bevorzugt umfasst die Komponente B) mindestens eine Komponente B2). Geeignete anionisch hydrophilierende Verbindungen als Komponente B2) enthalten bevorzugt eine Sulfonsäure- oder Sulfonatgruppe, besonders bevorzugt eine Natriumsulfonatgruppe. Geeignete anionisch hydrophilierende Verbindungen als Komponente B2) sind insbesondere die Alkalimetallsalze der Mono- und Diaminosulfonsäuren. Beispiele solcher anionischen Hydrophilierungsmittel sind Salze der 2-(2-Aminoethylamino)ethansulfonsäure, Ethylendiamin-propyl- oder -butylsulfonsäure, 1,2- oder 1,3-Propylendiamin-β-ethylsulfonsäure oder Taurin. Weiterhin kann das Salz der Cyclohexylaminopropansulfonsäure (CAPS) aus WO-A 01/88006 als anionisches Hydrophilierungsmittel verwendet werden.

Besonders bevorzugte anionische Hydrophilierungsmittel B2) sind solche, die Sulfonatgruppen als ionische Gruppen und zwei Aminogruppen enthalten, wie die Salze der 2-(2-Aminoethylamino)ethylsulfonsäure und 1,3-Propylendiamin-β-ethylsulfonsäure.

Besonders bevorzugt enthalten die erfindungsgemäß verwendeten Polyurethane mindestens eine Sulfonatgruppe.

Gegebenenfalls kann die anionische Gruppe in der Komponente B2) auch eine Carboxylat bzw. Carbonsäuregruppe sein. Die Komponente B2) wird dann bevorzugt aus Diaminocarbonsäuren ausgewählt. Diese Ausführungsform ist allerdings weniger bevorzugt, da Carbonsäure-basierende Komponenten B2) in höheren Konzentrationen eingesetzt werden müssen.

Zur Hydrophilierung können auch Mischungen aus anionischen Hydrophilierungsmitteln B2) und nichtionischen Hydrophilierungsmitteln A4) verwendet werden.

In einer bevorzugten Ausführungsform zur Herstellung der speziellen Polyurethan-Dispersionen werden die Komponenten A1) bis A4) und B1) bis B2) in den folgenden Mengen eingesetzt, wobei sich die Einzelmengen stets zu 100 Gew.-% addieren:
5 bis 40 Gew.-% Komponente A1),
55 bis 90 Gew.-% A2),
0,5 bis 20 Gew.-% Summe der Komponenten A3) und/oder B1)

0,1 bis 25 Gew.-% Summe der Komponenten A4) und/oder B2), wobei bezogen auf die Gesamtmengen der Komponenten A1) bis A4) und B1) bis B2) besonders bevorzugt 0,1 bis 5 Gew.-% an anionischen bzw. potentiell anionischen Hydrophilierungsmitteln B2) verwendet werden.

In einer besonders bevorzugten Ausführungsform zur Herstellung der speziellen Polyurethan-Dispersionen werden die Komponenten A1) bis A4) und B1) bis B2) in den folgenden Mengen eingesetzt, wobei sich die Einzelmengen stets zu 100 Gew.-% aufaddieren:
5 bis 35 Gew.-% Komponente A1),
60 bis 90 Gew.-% A2),
0,5 bis 15 Gew.-% Summe der Komponenten A3) und/oder B1)
0,1 bis 15 Gew.-% Summe der Komponenten Komponente A4) und/oder B2), wobei bezogen auf die Gesamtmengen der Komponenten A1) bis A4) und B1) bis B2) besonders bevorzugt 0,2 bis 4 Gew.-% an anionischen bzw. potentiell anionischen Hydrophilierungsmitteln B2) verwendet werden.

In einer ganz besonders bevorzugten Ausführungsform zur Herstellung der speziellen Polyurethan-Dispersionen werden die Komponenten A1) bis A4) und B1) bis B2) in den folgenden Mengen eingesetzt, wobei sich die Einzelmengen stets zu 100 Gew.-% aufaddieren:
10 bis 30 Gew.-% Komponente A1),
65 bis 85 Gew.-% A2),
0,5 bis 14 Gew.-% Summe der Komponenten A3) und/oder B1)
0,1 bis 13,5 Gew.-% Summe der Komponenten A4) und/oder B2), wobei bezogen auf die Gesamtmengen der Komponenten A1) bis A4) und B1) bis B2) besonders bevorzugt 0,5 bis 3,0 Gew.-% an anionischen bzw. potentiell anionischen Hydrophilierungsmitteln aus B2) verwendet werden.

Die Herstellung der Polyurethan-Dispersionen kann in einer oder mehreren Stufe/-n in homogener oder bei mehrstufiger Umsetzung, teilweise in disperser Phase durchgeführt werden. Nach vollständig oder teilweise durchgeführter Polyaddition aus A1) bis A4) erfolgt bevorzugt ein Dispergier-, Emulgier- oder Lösungsschritt. Im Anschluss erfolgt gegebenenfalls eine weitere Polyaddition oder Modifikation in disperser Phase.

Dabei können alle aus dem Stand der Technik bekannten Verfahren wie z. B. Prepolymer-Mischverfahren, Acetonverfahren oder Schmelzdispergierverfahren verwendet werden. Bevorzugt wird nach das Aceton-Verfahren angewandt.

Für die Herstellung nach dem Aceton-Verfahren werden üblicherweise die Bestandteile A2) bis A4) und die Polyisocyanatkomponente A1) zur Herstellung eines isocyanatfunktionellen Polyurethan-Prepolymers ganz oder teilweise vorgelegt und gegebenenfalls mit einem mit Wasser mischbaren aber gegenüber Isocyanatgruppen inerten Lösungsmittel verdünnt und auf Temperaturen im Bereich von 50 bis 120°C aufgeheizt. Zur Beschleunigung der Isocyanatadditionsreaktion können die in der Polyurethan-Chemie bekannten Katalysatoren eingesetzt werden.

Geeignete Lösungsmittel sind die üblichen aliphatischen, ketofunktionellen Lösemittel wie Aceton, 2-Butanon, die nicht nur zu Beginn der Herstellung, sondern gegebenenfalls in Teilen auch später zugegeben werden können. Bevorzugt sind Aceton und 2-Butanon, besonders bevorzugt ist Aceton. Auch die Zugabe anderer Lösemittel ohne isocyanatreaktive Gruppen ist möglich, jedoch nicht bevorzugt.

Anschließend werden die gegebenenfalls zu Beginn der Reaktion noch nicht zugegebenen Bestandteile von A1) bis A4) zudosiert.

Bei der Herstellung des Polyurethan-Prepolymeren aus A1) bis A4) beträgt das Stoffmengenverhältnis von Isocyanat-Gruppen zu isocyanatreaktiven Gruppen im allgemeinen 1,05 bis 3,5, bevorzugt 1,1 bis 3,0, besonders bevorzugt 1,1 bis 2,5.

Die Umsetzung der Komponenten A1) bis A4) zum Prepolymer erfolgt teilweise oder vollständig, bevorzugt aber vollständig. Es werden so Polyurethan-Prepolymere, die freie Isocyanatgruppen enthalten, in Substanz oder in Lösung erhalten.

Im Neutralisationsschritt zur teilweisen oder vollständigen Überführung potentiell anionischer Gruppen in anionische Gruppen werden Basen wie tertiäre Amine, z.B. Trialkylamine mit 1 bis 12, bevorzugt 1 bis 6 C-Atomen, besonders bevorzugt 2 bis 3 C-Atomen in jedem Alkylrest oder ganz besonders bevorzugt Alkalimetallbasen wie die entsprechenden Hydroxide eingesetzt. Die Verwendung von organischen Aminen ist nicht bevorzugt.

Als Neutralisationsmittel werden bevorzugt anorganische Basen, wie wässrige Ammoniaklösung oder Natrium- bzw. Kaliumhydroxid einsetzbar. Bevorzugt sind Natriumhydroxid und Kaliumhydroxid.

Die Stoffmenge der Basen beträgt 50 und 125 mol%, bevorzugt zwischen 70 und 100 mol% der Stoffmenge der zu neutralisierenden Säuregruppen. Die Neutralisation kann auch gleichzeitig mit der Dispergierung erfolgen, in dem das Dispergierwasser bereits das Neutralisationsmittel enthält. Im Anschluss wird in einem weiteren Verfahrensschritt, falls noch nicht oder nur teilweise geschehen, das erhaltene Prepolymer mit Hilfe von aliphatischen Ketonen wie Aceton oder 2-Butanon gelöst.

Die Umsetzung der Komponenten A1) bis A4) zum Prepolymer erfolgt teilweise oder vollständig, bevorzugt aber vollständig. Es werden so Polyurethan-Prepolymere, die freie Isocyanatgruppen enthalten, in Substanz oder in Lösung erhalten.

Bei der Kettenverlängerung in Stufe B) werden NH₂- und/oder NH-funktionelle Komponenten mit den noch verbliebenen Isocyanatgruppen des Prepolymers umgesetzt. Bevorzugt wird die Kettenverlängerung/-terminierung vor der Dispergierung in Wasser durchgeführt.

Geeignete Komponenten B) zur Kettenverlängerung sind insbesondere organische Di- oder Polyamine B1) wie beispielsweise Ethylendiamin, 1,2- und 1,3-Diaminopropan, 1,4-Diaminobutan, 1,6-Diaminohexan, Isophorondiamin, Isomerengemisch von 2,2,4- und 2,4,4-Trimethylhexamethylendiamin, 2-Methylpentamethylendiamin, Diethylentriamin, Diaminodicyclohexylmethan und/oder Dimethylethylendiamin.

Darüber hinaus können auch Verbindungen B1), die neben einer primären Aminogruppe auch sekundäre Aminogruppen oder neben einer Aminogruppe (primär oder sekundär) auch OH-Gruppen aufweisen, eingesetzt werden. Beispiele hierfür sind primäre/sekundäre Amine, wie Diethanolamin, 3-Amino-1-methylaminopropan, 3-Amino-1-ethylaminopropan, 3-Amino-1-cyclohexylaminopropan, 3-Amino-1-methylaminobutan, Alkanolamine wie N-Aminoethylethanolamin, Ethanolamin, 3-Aminopropanol, Neopentanolamin zur Kettenverlängerung bzw. -terminierung eingesetzt werden.

Zur Kettenterminierung werden üblicherweise Amine B1) mit einer gegenüber Isocyanaten reaktiven Gruppe wie Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, bzw. geeignete substituierte Derivate davon, Amidamine aus diprimären Aminen und Monocarbonsäuren, Monoketim von diprimären Aminen, primär/tertiäre Amine, wie N,N-Dimethylaminopropylamin verwendet.

Werden zur Kettenverlängerung anionische Hydrophilierungsmittel entsprechend der Definition B2) mit NH₂- oder NH-Gruppen eingesetzt, erfolgt die Kettenverlängerung der Prepolymere bevorzugt vor der Dispergierung.

Der Kettenverlängerungsgrad, also das Äquivalentverhältnis von NCO-reaktiven Gruppen der zur Kettenverlängerung und Kettenterminierung eingesetzten Verbindungen zu freien NCO-Gruppen des Prepolymers, liegt im allgemeinen zwischen 40 und 150 %, bevorzugt zwischen 50 und 110 %, besonders bevorzugt zwischen 60 und 100 %.

Die aminischen Komponenten B1) und B2), können gegebenenfalls in wasser- oder lösemittelverdünnter Form im erfindungsgemäßen Verfahren einzeln oder in Mischungen eingesetzt werden, wobei grundsätzlich jede Reihenfolge der Zugabe möglich ist.

Wenn Wasser oder organische Lösemittel als Verdünnungsmittel mit verwendet werden, so beträgt der Verdünnungsmittelgehalt in der in B) eingesetzten Komponente zur Kettenverlängerung bevorzugt 40 bis 95 Gew.-%.

Die Dispergierung erfolgt bevorzugt im Anschluss an die Kettenverlängerung. Dazu wird das gelöste und kettenverlängerte Polyurethanpolymer gegebenenfalls unter starker Scherung, wie z.B. starkem Rühren, entweder in das Dispergierwasser eingetragen oder es wird umgekehrt das Dispergierwasser zu den kettenverlängerte Polyurethanpolymerlösungen gerührt. Bevorzugt wird das Wasser in das gelöste kettenverlängerte Polyurethanpolymer gegeben.

Das in den Dispersionen nach dem Dispergierschritt noch enthaltene Lösemittel wird üblicherweise anschließend destillativ entfernt. Eine Entfernung bereits während der Dispergierung ist ebenfalls möglich.

Der Restgehalt an organischen Lösemitteln in den so hergestellten Polyurethan-Dispersionen beträgt typischerweise weniger als 10 Gew.-%, bevorzugt weniger als 3 Gew.-%, bezogen auf die gesamte Dispersion.

Der pH-Wert der erfindungsgemäß verwendeten wässrigen Polyurethan-Dispersionen beträgt typischerweise weniger als 8,0, bevorzugt weniger als 7,5 und liegt besonders bevorzugt zwischen 5,5 und 7,5.

Die erfindungsgemäße Sonnenschutz-Zusammensetzung enthält bevorzugt 0,1 bis 20 Gewichts-% des oben beschriebenen Polyurethans und insbesondere 0,5 bis 10 Gewichts-% jeweils bezogen auf das Gesamtgewicht der Zusammensetzung.

Die erfindungsgemäße Sonnenschutz-Zusammensetzung verbleibt nach dem Auftrag naturgemäß zumindest zum Teil auf der Haut, und unterscheidet sich somit beispielsweise von kosmetischen Produkten, die nach Verwendung auf der Haut entfernt werden, wie zum Beispiel kosmetischen Gesichtsmasken und Reinigungsprodukte, wie Seifen etc. Die erfindungsgemäße Sonnenschutz-Zusammensetzung umfasst des Weiteren im Allgemeinen auch keine Haarpflegemittel, Make-up-Zusammensetzungen, wie Schminke etc., keine Make-up-Lippenstifte und keine Nagellacke oder dergleichen.

In Sinne der vorliegenden Erfindungen unterscheiden sich die Sonnenschutz-Zusammensetzungen insbesondere durch ihre Konsistenz, d.h. Creme (viskos), Lotion und Milch (fließfähig), Gel (halbfest) sowie dünnflüssige Formulierungen wie zum Beispiele Spray, Balsam und wässrige Lösungen.

Die Sonnenschutz-Zusammensetzungen können beispielweise in Form von Öl-in-Wasser-, Wasser-in-Öl-, Wasser-in-Silikon-, Silikon-in-Wasser, Öl-in-Wasser-in-Öl-, Wasser-in-Öl-in-Wasser-Emulsion vorliegen.

Die Sonnenschutz-Zusammensetzung können ferner mit einem Treibgas aufgeschäumt werden.

Die oben geschriebenen Emulsionen können beispielsweise durch O/W-, W/O- oder W/Si-Emulgatoren, Verdicker, beispielweise Hydrodispersion, oder Feststoffe, beispielweise Pickeringemulsion, stabilisiert sein.

Die Sonnenschutz-Zusammensetzungen können einen oder mehrere Emulgatoren bzw. oberflächenaktive Mittel enthalten. So enthalten insbesondere erfindungsgemäße Öl-in-Wasser-Emulsionen (O/W) mindestens einen Emulgator mit einem HLB-Wert > 7 und gegebenenfalls einen Coemulgator.

O/W Emulgatoren können vorteilhaft gewählt werden aus der Gruppe der nichtionischen, anionischen, kationischen oder amphoteren Emulgatoren.

Unter den nichtionischen Emulgatoren befinden sich beispielsweise Partialfettsäureester und Fettsäureester mehrwertiger Alkohole und deren ethoxylierte Derivate, ethoxylierte Fettalkohole und Fettsäuren, ethoxylierte Fettamine, Fettsäureamide, Fettsäurealkanolamide, Alkylphenolpolyglycolether, z.B. Triton® X, und/oder ethoxylierte Fettalkoholether.

Besonders vorteilhafte nichtionische O/W-Emulgatoren sind ethoxylierte Fettalkohole oder Fettsäuren, bevorzugt PEG-100-Stearat, PEG-40-Stearat, PEG-50 Stearat, Ceteareth-20, Ceteth-20, Steareth-20, Ceteareth-12, Ceteth-12, Steareth-12, Ester aus Mono-, Oligo- oder Polysacchariden mit Fettsäuren, bevorzugt Cetearylglucosid, Methylglucosedistearat, Glycerylmonostearate (selbstemulgierend), Sorbitanester wie zum Beispiel Sorbitanstearate (Tween® 20 und Tween® 60 der FirmaCroda), Sorbitanpalmitate (Span® 40, Croda), Glycerylstearylcitrate, Sucroseester, zum Beispiel Sucrosestearate, PEG-20-Methylglucose-sequistearat-Dicarbonsäureester von Fettalkohol (Dimyristyl tartrate).

Vorteilhafte anionische Emulgatoren sind Seifen, z. B. Natrium- oder Triethanolamin-Salze der Stearin- oder Palmitinsäure), Ester der Zitronensäure wie Glycerylstearatcitrat, Fettalkoholsulfate sowie Mono-, Di- und Trialkylphosphorsäureester und deren Ethoxylate.

Unter den kationischen Emulgatoren befinden sich quaternäre Ammoniumverbindungen mit einem langkettigen aliphatischen Rest, z.B. Distearyldiammoniumchlorid.

Unter den amphoteren Emulgatoren befinden sich beispielsweise Alkylamininoalkancarbonsäuren, Betaine, Sulfobetaine und/oder Imidazolinderivate.

Weiterhin gibt es natürlich vorkommende Emulgatoren, beispielsweise Bienenwachs, Wollwachs, Lecithin und/oder Sterole.

Als geeignete Coemulgatoren für die erfindungsgemäßen O/W-Emulsionen können Fettalkohole mit 8 bis 30 Kohlenstoffatomen, Monoglycerinester gesättigter oder ungesättigter, verzweigter oder unverzweigter Alkancarbonsäuren mit einer Kettenlänge von 8 bis 24 Kohlenstoffatomen, insbesondere 12 bis 18 Kohlenstoffatomen, Propylenglycolester gesättigter oder ungesättigter, verzweigter oder unverzweigter Alkancarbonsäuren mit einer Kettenlänge von 8 bis 24 Kohlenstoffatomen, insbesondere 12 bis 18 Kohlenstoffatomen, sowie Sorbitanester gesättigter oder ungesättigter, verzweigter oder unverzweigter Alkancarbonsäuren mit einer Kettenlaenge von 8 bis 24 Kohlenstoffatomen, insbesondere 12 bis 18 Kohlenstoffatomen, eingesetzt werden.

Besonders vorteilhafte Coemulgatoren sind Glycerylmonostearat, Glycerylmonooleat, Diglycerylmo-nostearat, Sorbitanmonoisostearat, Saccharosedistearat, Cetylalkohol, Stearylalkohol, Behenylalkohol, Isobehenylalkohol und Polyethylenglycol(2)stearylether (Steareth-2).

Es kann im Sinn der vorliegenden Erfindung vorteilhaft sein, weitere Emulgatoren zu verwenden. So kann beispielsweise die Wasserfestigkeit der erfindungsgemäßen Zubereitungen auf diese Weise weiter erhöht werden. Geeignete Emulgatoren sind z.B. Alkylmethiconcopolyole und AlkylDimethiconcopolyole, insbesondere Cetyldimethiconcopolyol, Laurylmethiconcopolyol, W/O-Emulgatoren wie Sorbitanstearat, Glycerylstearat, Glycerolstearat, Sorbitanoleat, Lecithin, Glyceryl-isostearat, Polyglyceryl-3-oleat, Polyglyceryl-3-diisostearat, PEG-7-hydriertes Ricinusoel, Polyglyce-ryl-4-isostearat, Acrylat/C10-30-Alkylacrylat-Crosspolymer, Sorbitanisostearat, Poloxamer 101, Poly-glyceryl-2-dipolyhydroxystearat, Polyglyceryl-3-diisostearat, Polyglyceryl-4-dipolyhydroxystearat, PEG-30-dipolyhydroxystearat, Diisostearoylpolyglyceryl-3 -diisostearat, Glycoldistearat und Polygly-ceryl-3-dipolyhydroxystearat.

Die erfindungsgemäßen Zusammensetzungen, insbesondere die O/W-Zusammensetzungen, können vorteilhaft Verdicker der Wasserphase enthalten.

Vorteilhafte Verdicker sind beispielsweise vernetzte oder nichtvernetzte Acrylsäure oder Methacrylsäure-Homo- oder Copolymere. Hierzu gehören insbesondere vernetzte Hompolymere von Methacrylsäure oder Acrylsäure, Copolymerisate aus Acrylsäure und/oder Methacrylsäure und Monomeren, die von anderen Acryl- oder Vinylmonomeren abgeleitet sind, wie C10-30 Alkylacrylate, C10-30-Alkylmethacrylate, Vinylacetat und Vinylpyrrolidone.

Verdickende Polymere natürlicher Herkunft sind beispielweise auf Cellulosebasis, Guargummi, Xanthan, Scleroglucan, Gellangummi, Rhamsan und Karayagummi, Alginate, Maltodextrin, Stärke und ihre Derivate, Johannisbrotkernmehl, Hyaluronsäure, Carrageenan, nichtionische, anionische, kationische oder amphotere assoziative Polymere z.B. auf Basis von Polyethylenglycole und ihre Derivate, oder Polyurethane, vernetzte oder nichtvernetzte Homopolymere oder Copolymere auf Basis von Acrylamid oder Methacrylamid, wie Homopolymere von 2-Acrylamido-2-methylpropansulfonsaeure, Copolymere von Acrylamid oder Methacrylamid und Methacryloyloxyethyltrimethylammoniumchlorid oder Copolymere von Acrylamid und 2-Acrylamido-2-methylpropansulfonsäure.

Besondere vorteilhafte Verdicker sind verdickende Polymere natürlicher Herkunft, vernetzte Acrylsäure- oder Methacrylsäure-Homo- oder Copolymere und vernetzte Copolymere von 2-Acrylamido-2-methylpropansulfonsäure. Ganz besonders vorteilhafte Verdicker sind Xanthangummi, wie die unter den Bezeichnungen Keltrol® und Kelza® von der Firma CP Kelco angebotenen Produkte oder die Produkte der Firma RHODIA mit der Bezeichnung Rhodopol und Guargummi, wie die unter der Bezeichnung Jaguar® HP105 von der Firma RHODIA erhältlichen Produkte.

Ganz besonders vorteilhafte Verdicker sind auch vernetzte Homopolymere von Methacrylsäure oder Acrylsäure, die von der Firma Lubrizol unter den Bezeichnungen Carbopol® 940, Carbopol® 941, Carbopol® 980, Carbopol® 981, Carbopol® ETD 2001, Carbopol® EDT 2050, Carbopol® 2984, Carbo-pol® 5984 und Carbopol® Ultrez 10, von der Firma 3V, die unter den Bezeichnungen Synthalen® K, Synthalen® L und Synthalen® MS im Handel erhältlich sind.

Ganz besonders vorteilhafte Verdicker sind vernetzte Copolymere von Acrylsäure oder Methacrylsäure und einem C10-30-Alkylacrylat oder C10-30-Alkylmethacrylat und Coplymere von Acrylsäure oder Methacrylsäure und Vinylpyrrolidone. Solche Copolymere sind beispielsweise von der Firma Lubrizol unter den Bezeichnungen Carbopol® 1342, Carbopol® 1382, Pemulen® TR1 oder Pemulen® TR2 und von der Firma Ashland unter den Bezeichnungen Ultrathix® P-100 (INCI: Acrylic Acid/VP Crosspolymer) im Handel erhältlich.

Ganz besondere vorteilhafte Verdicker sind vernetzte Copolymere von 2-Acrylamido-2-methylpropansulfonsäure. Solche Copolymere sind beispielsweise von der Firma Clariant unter den Bezeichnungen Aristoflex® AVC (INCI: Ammonium Acryloyldimethyltaurate/VP Copolymer) erhältlich

Diese Verdicker sind im Allgemeinen in einer Konzentration von etwa 0 bis 2 Gew.-%, vorzugsweise 0 bis 1 Gew.-%, jeweils bezogen auf das Gesamtgewicht der erfindungsgemäßen Zusammensetzung, vorhanden.

Weitere erfindungsgemäße Zusammensetzungen können Wasser-in-Öl- oder Wasser-in-Silikon-Emulsionen sein. Bevorzugt sind Wasser-in-Öl- (W/O) oder Wasser-in-Silikon-Emulsionen (W/Si), die ein oder mehrere Silikonemulgatoren (W/S) mit einem HLB-Wert ≤ 8 oder ein oder mehrere W/O-Emulgatoren mit einem HLB-Wert < 7 und gegebenenfalls ein oder mehrere O/W-Emulgatoren mit einem HLB-Wert > 10 enthalten.

Die Silikonemulgatoren können vorteilhaft aus der Gruppe enthaltend Alkyldimethiconcopolyole wie z. B. Cetyl PEG/PPG 10/1 Dimethiconcopolyol (ABIL® EM 90 der Fa. Evonik) oder Lauryl PEG/PPG- 18/18 Dimethicone (Dow Corning® 5200 der Fa. Dow Corning Ltd.) und Dimethiconeco-polyole wie z. B. PEG-10 Dimethicone (KF-6017 der Fa. Shin Etsu), PEG/PPG- 18/18 Dimethicone (Dow Corning 5225C der Fa. Dow Corning Ltd.), PEG/PPG-19/19 Dimethicone (Dow Corning BY-11 030 der Fa. Dow Corning Ltd.) oder Trimethylsilylamodimethicone gewählt werden.

Die W/O-Emulgatoren mit einem HLB-Wert < 7 können vorteilhaft aus der folgenden Gruppe ausgewählt werden Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 bis 18 C-Atomen, Diglycerinester gesättigter und/oder ungesättigter, verzweigter und/oder unverzweigter Alkancarbonsäuren einer Kettenlänge von 8 bis 24, insbesondere 12 bis 18 C-Atomen, Monoglycerinether gesättigter und/oder ungesättigter, verzweigter und/oder unverzweigter Alkohole einer Kettenlänge von 8 bis 24, insbesondere 12 bis 18 C-Atomen, Diglycerinether gesättigter und/oder ungesättigter, verzweigter und/oder unverzweigter Alkohole einer Kettenlänge von 8 bis 24, insbesondere 12 bis 18 C-Atomen, Propylenglycolester gesättigter und/oder ungesättigter, verzweigter und/oder unverzweigter Alkancarbonsäuren einer Kettenlänge von 8 bis 24, insbesondere 12 bis 18 C-Atomen sowie Sorbitanester gesättigter und/oder ungesättigter, verzweigter und/oder unverzweigter Alkancarbonsäuren einer Kettenlänge von 8 bis 24, insbesondere 12 bis 18 C-Atomen.

Besonders vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat und Glycerylmonocaprylat.

Weitere mögliche W/O-Emulgatoren werden gewählt aus der Gruppe der Verbindungen Polyglyceryl-2-dipolyhydroxystearat, PEG-30 Dipolyhydroxystearat, Cetyl Dimethicon Copolyol und Polyglyceryl- Diisostearat.

Die O/W-Emulgatoren mit einem HLB-Wert > 10 können vorteilhaft aus der Gruppe enthaltend Lecithin, Trilaureth-4-Phosphat, Polysorbate-20, Polysorbate-60, PEG-22-Dodecylglycol Copolymer, Sucrosestearat und Sucroselaurat gewählt werden.

Zur Stabilisierung der erfindungsgemäßen W/O-Emulsion gegen Sedimentierung oder Flockung der Wassertröpfchen kann vorteilhaft ein Ölverdicker eingesetzt werden.

Besonders vorteilhafte Ölverdicker sind organomodifizierte Tone wie organomodifizierte Bentonite (Bentone® 34 der Firma Elementis) organomodifizierte Hectorite (Bentone® 27 und Bentone® 38 der Firma Elementis) oder organomodifizierte Montmorillonit, hydrophobe pyrogene Kieselsäure, wobei die Silanolgruppen mit Trimethylsiloxygruppen subtituiert sind (AEROSIL® R812 der Firma Degussa) oder mit Dimethylsiloxygruppen oder Polydimethylsiloxan (AEROSIL® R972, AEROSIL® R974 von Degussa, CAB-O-SIL® TS-610, "CAB-O-SIL® TS-720 von Cabot), Magnesium- oder Aluminiumste-arat, oder Styrol-Copolymere wie zum Beispiel Styrol-Butadien-Styrol, Styrol-Isopropen-Styrol, Sty-rol-Ethylen/Buten-Styrol oder Styrol-Ethylen/Propen-Styrol.

Das Verdickungsmittel für die Fettphase kann in einer Menge von 0,1 bis 5 Gew.-%, bevorzugt 0,4 bis 3 Gew.-%, jeweils bezogen auf das Gesamtgewicht der Emulsion, enthalten sein.

Die wässrige Phase kann ferner Stabilisierungsmittel enthalten. Das Stabilisierungsmittel kann beispielsweise Natriumchlorid, Magnesiumchlorid oder Magnesiumsulfat und deren Gemischen sein.

Öle können in W/O-, W/Si- und O/W-Emulsionen eingesetzt werden.

Wenn vorhanden, kann die Fettphase der erfindungsgemäßen Zusammensetzung ein nicht flüchtiges Öl und/oder flüchtige Öle und Wachse enthalten. Die O/W- Zusammensetzung enthält vorteilhaft 0,01 bis 45 Gew.-%, besonders vorteilhaft 0,01 bis 20 Gew.-%, Öle, jeweils bezogen auf das Gesamtgewicht der Zusammensetzung. Die W/O oder W/Si-Zusammensetzung enthält vorteilhaft mindestens 20 Gew.-% Öle, bezogen auf das Gesamtgewicht der Zusammensetzung.

Das nicht flüchtige Öl wird vorteilhaft gewählt aus der Gruppe von mineralischer, tierischer, pflanzlicher oder synthetischer Herkunft, polaren oder unpolaren Ölen und deren Gemischen.

Die Lipidphase der erfindungsgemäßen kosmetischen oder dermatologischen Emulsionen kann vor-teilhaft gewählt werden aus der Substanzgruppe enthaltend Mineralöle, Mineralwachse, polare Öle, wie Triglyceride der Caprin- oder der Caprylsäure, ferner natürliche Öle wie z.B. Rizinusöl; Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren; Alkylbenzoate; Silikonöle wie Dimethylpolysiloxane, Diethylpolysiloxane, Diphenylpolysiloxane sowie Mischformen daraus.

Die polaren Öle werden vorteilhaft gewählt aus der Gruppe aus Estern aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, Estern aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe bestehend aus Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, Isotridecylisononanoat, 2-Ethylhexylpalmitat, 2-Ethylhexyllaurat, 2-Ethylhexylisostearat, 2-Hexyldecylstearat, 2-Octyldodecylpalmitat, 2-Ethylhexylcocoat, Oleyloleat, Oleylerucat, Erucyloleat, Erucylerucat Di-caprypyp Carbonat (Cetiol® CC) und Cocoglyceride (Myritol® 331) sowie synthetische, halbsyntheti-sche und natürliche Gemische solcher Ester, z.B. Jojobaöl, Alkylbenzoate C12-15-Alkylbenzoat (Finsolv® TN von Finetex) oder 2-Phenylethyl benzoate (X-Tend® 226 von Ashland), Lecithine und den Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen ausgewählt werden. Beipielweise können die Fettsäuretriglyceride gewählt werden aus der Gruppe von Cocoglycerid, Olivenöl, Sonnenblumenöl, Sojaöl, Erdnussöl, Rapsöl, Mandelöl, Palmöl, Kokosöl, Rizinusöl, Weizenkeimöl, Traubenkernöl, Distelöl, Nachtkerzenöl, Macadamianussöl, Aprikosenkernöl, Avocadoöl und dergleichen mehr, der Dialkylether und Dialkylcarbonate, vorteilhaft sind z.B. Dicaprylylether (Cetiol® OE der Firma BASF) und/oder Dicaprylylcarbonat (beispielsweise Cetiol® CC der Firma BASF), gesättigten oder ungesättigten, verzweigten oder unverzweigten Alkohole, wie zum Beispiel Octyldodecanol.

Das nicht flüchtige Öl kann ebenfalls vorteilhaft auch ein unpolares Öl sein, welche gewählt wird aus der Gruppe der verzweigten und unverzweigten Kohlenwasserstoffe, insbesondere Mineralöl, Vaselineöl, Paraffinöl, Squalan und Squalen, Polyolefine beispielweise Polydecene, hydrogenierte Polyisobutene, C13-16 Isoparaffin und Isohexadecan.

Das unpolare nicht flüchtige Öl kann unter den nicht flüchtigen Silikonölen ausgewählt werden. Von den nicht flüchtigen Silikonölen können die Polydimethylsiloxane (PDMS), die gegebenenfalls phenyliert sind, wie die Phenyltrimethicon, oder gegebenenfalls substituiert sind mit aliphatischen und/oder aromatischen Gruppen oder mit funktionellen Gruppen, beispielsweise Hydroxygruppen, Thiolgruppen und/oder Aminogruppen; mit Fettsäuren, Fettalkoholen oder Polyoxyalkylenen modifizierte Polysiloxane und deren Gemische angegeben werden.

Besondere vorteilhafte Öle sind 2-Ethylhexylisostearat, Octyldodecanol, Isotridecylisononanoat, Isoeicosan, 2-Ethylhexylcocoat, C12-15 Alkylbenzoat, Caprylic/Capric Triglycerid, Dicaprylylether, Mineral Öl, Dicaprylyl Carbonate, Cocoglyceride, Butylene Glykol Dicarprylate/Dicaprate, Hydroge-nated Polyisobutene, Cetaryl Isononanoate, Isodecyl Neopentanoate, Squalan, C13-16 Isoparaffin.

Die erfindungsgemäße Zusammensetzung kann ferner ein Wachs enthalten. Im Sinne der vorliegenden Schrift ist ein Wachs als lipophile Fettsubstanz definiert, die bei Raumtemperatur; d.h. 25 °C, fest ist und bei einer Schmelztemperatur von 30 bis 200 °C eine reversible Zustandsänderung fest/flüssig zeigt. Oberhalb des Schmelzpunktes wird das Wachs niedrigviskos und mischbar mit Ölen.

Das Wachs wird vorteilhaft gewählt aus der Gruppen von natürlichen Wachsen wie beispielweise Baumwollwachs, Carnaubawachs, Candelillawachs, Espartoawachs, Japanwachs, Montanwachs, Zu-ckerrohrwachs, Bienenwachs, Wollwachs, Schellack, Mikrowachse, Ceresin, Ozokerit, Ouricuri-wachs, Korkfaserwachs, Lignitwachse, Berrenwachs, Sheabutter oder synthetische Wachse wie Paraffinwachse, Polyethylenwachse, durch Fischer-Tropsch-Synthese hergestellte Wachse, hydrierte Öle, Fettsäureester und Glyceride, die bei 25 °C fest sind, Silikonwachse und Derivaten (lkylderivate, Alkoxyderivate und/oder Ester von Polymethylsiloxan) und deren Gemischen. Die Wachse können in Form von stabilen Dispersionen von kolloidalen Wachspartikeln vorliegen, die nach bekannten Verfahren hergestellt werden können, beispielsweise gemäß "Microemulsions Theory and Practice", L.M. Prince Ed., Academic Press (1977), Seiten 21 bis 32.

Die Wachse können in Mengen von 0 bis 10 Gew.-%, vorzugsweise 0 bis 5 Gew.-% jeweils bezogen auf das Gesamtgewicht der Zusammensetzung enthalten sein.

Die erfindungsgemäße Zusammensetzung kann ferner ein flüchtiges Öl enthalten, das aus der Gruppe von flüchtigen Kohlenwasserstoffölen, silikonierten Ölen oder fluorierten Ölen ausgewählt wird.

Das flüchtige Öl kann in einer Menge von 0 bis 25 Gew.-%, vorzugsweise 0 bis 20 Gew.-%, noch bevorzugter 0 bis 15 Gew.-%, jeweils bezogen auf das Gesamtgewicht der Emulsion, enthalten sein.

Im Sinne der vorliegenden Schrift ist ein flüchtiges Öl ein Öl, das im Kontakt mit der Haut bei Raumtemperatur und Atmosphärendruck in weniger als einer Stunde verdampft. Das flüchtige Öl ist bei Raumtemperatur flüssig und bei Raumtemperatur und Atmosphärendruck einen Dampfdruck von 0,13 bis 40 000 Pa (10⁻³ bis 300 mg Hg), vorzugsweise 1,3 bis 13 000 Pa (0,01 bis 100 mm Hg) und besonders bevorzugt 1,3 bis 13 00 Pa (0,01 bis 10 mm Hg), und einen Siedepunkt von 150 bis 260 °C und vorzugsweise 170 bis 250 °C besitzt.

Unter einem Kohlenwasserstofföl wird ein Öl verstanden, das im Wesentlichen aus Kohlenstoffatomen und Wasserstoffatomen und gegebenenfalls Sauerstoffatomen oder Stickstoffatomen gebildet wird und keine Siliziumatome oder Fluoratome enthält, wobei es auch aus Kohlenstoffatomen und Wasserstoffatomen bestehen kann; es kann Estergruppen, Ethergruppen, Aminogruppen oder Amid-gruppen enthalten.

Unter einem silikonierten Öl wird ein Öl verstanden, das mindestens ein Siliziumatom und insbesondere Si-O-Gruppen enthält.

Unter einem fluorierten Öl ist ein Öl zu verstehen, das mindestens ein Fluoratom enthält.

Das erfindungsgemäß flüchtige Kohlenwasserstofföl kann unter den Kohlenwasserstoffölen mit einem Flammpunkt von 40 bis 102 °C, vorzugsweise 40 bis 55 °C und noch bevorzugter 40 bis 50 °C ausgewählt werden.

Beispielweise sind die flüchtigen Kohlenwasserstofföle solche mit 8 bis 16 Kohlenstoffatomen und deren Gemische, insbesondere verzweigte C8-16-Alkane, wie die Isoalkane, die auch als Isoparaffine bezeichnet werden, mit 8 bis 16 Kohlenstoffatomen, Isododecan, Isodecan, Isohexadecan und bei-spielsweise die Öle, die unter den Handelsnamen Isopars® oder Permetyls® angeboten werden; und die verzweigten C8-16-Ester, wie Isohexylneopentanoat und deren Gemische.

Besonders vorteilhaft sind die flüchtigen Kohlenwasserstofföle wie Isododecan, Isodecan und Isohe-xadecan.

Das erfindungsgemäß flüchtige silikonierte Öl kann unter den silikonierten Ölen mit einem Flammpunkt von 40 bis 102 °C, vorzugsweise einem Flammpunkt über 55 °C und höchstens 95 °C und besonders bevorzugt im Bereich von 65 bis 95 °C ausgewählt werden.

Beispielweise sind die flüchtigen silikonierten Öle geradkettigen oder cyclischen Silikonöle mit 2 bis 7 Siliziumatomen genannt werden, wobei diese Silikone gegebenenfalls Alkyl- oder Alkoxygruppen mit 1 bis 10 Kohlenstoffatomen enthalten.

Besonders vorteilhaft sind die flüchtigen silikonierten Öle wie Octamethylcyclotetrasiloxan, Decamethylcyclopentasiloxan, Dodecamethylcyclohexasiloxan, Heptamethylhexyltrisiloxan, Heptamethyloctyltrisiloxan, Hexamethyldisiloxan, Octamethyltrisiloxan, Decamethyltetrasiloxan, Dodecamethylpentasiloxan und deren Gemische.

Das flüchtige fluorierte Öl besitzt im Allgemeinen keinen Flammpunkt.

Beispielweise sind die flüchtigen fluorierten Öle Nonafluorethoxybutan, Nonafluormethoxybutan, Decafluorpentan, Tetradecafluorhexan, Dodecafluorpentan und deren Gemische.

Das kosmetische akzeptable Medium der erfindungsgemäßen Zusammensetzung enthält Wasser und gegebenenfalls ein kosmetisch im Wasser mischbares geeignetes organisches Lösungsmittel.

Das verwendete Wasser in der erfindungsgemäßen Zusammensetzung kann ein Blütenwasser, reines demineralisiertes Wasser, Mineralwasser, Thermalwasser und/oder Meerwasser sein.

Im Fall einer O/W Zusammensetzung kann der Wasseranteil im Bereich von 40 bis 95 Gew.-%, bevorzugt im Bereich von 50 bis 90 Gew.-%, ganz bevorzugt im Bereich von 60 bis 80 Gew.-%, jeweils bezogen auf das Gesamtgewicht der Zusammensetzung, liegen. Im Fall einer W/O Zusammensetzung liegt der Wasseranteil im Bereich von 0 bis 60 Gew.-%, bevorzugt im Bereich von 10 bis 50 Gew.-%, ganz bevorzugt im Bereich von 30 bis 50 Gew.-%, jeweils bezogen auf das Gesamtgewicht der Zusammensetzung.

Die bevorzugten Lösungsmittel sind beispielsweise die aliphatischen Alkohole mit C1-4 Kohlenstoffatomen wie Ethanol und Isopropanol; Polyol und deren Derivate wie Propylenglykol, Dipropylenglykol, Butylen-1,3-glykol, Polypropylenglykol, Glykolether wie Alkyl (C1-4)ether von Mono-, Di- oder Tripropylenglykol oder Mono-; Di- oder Triethylenglykol, und deren Gemischen ausgewählt.

Der Mengenanteil des Lösungsmittels oder der Lösungsmittel in der erfindungsgemäßen Zusammensetzung kann beispielsweise im Bereich von 0 bis 25 Gew.-% und bevorzugt 0 bis 10 Gew.-%, jeweils bezogen auf das Gesamtgewicht der Zusammensetzung, liegen.

Die erfindungsgemäße Sonnenschutz-Zusammensetzung enthält einen oder mehrere Sonnenschutzfilter bzw. Sonnenschutzfilter-Substanzen bzw. Sonnenschutz vermittelnde Substanzen.

Bei den Sonnenschutzfiltern handelt es sich insbesondere um UV-Filter, welche Licht im UV-Wellenlängenbereich insbesondere von weniger als 400 nm filtern. Üblicherweise unterteilt man den UV-Wellenlängenbereich wie folgt:

| | |
|---|---|
| Nahes UV | Wellenlänge 400 bis 200 nm |
| UV-A | Wellenlänge 380 bis 315 nm |
| UV-B | Wellenlänge 315 bis 280 nm |
| UV-C | Wellenlänge 280 bis 100 nm |

| | |
|---|---|
| Fernes UV, Vakuumstrahlung | Wellenlänge 200 bis 10 nm |
| Extremes UV | Wellenlänge 31 bis 1 nm |

Die Menge der in der erfindungsgemäßen Sonnenschutz-Zusammensetzung verwendeten Sonnenschutzfilter, insbesondere UV-Filter, liegt zweckmäßig im Bereich von 0 bis 30 Gew.%, vorteilhaft 0 bis 20 Gew.%, besonders vorteilhaft 0 bis 10 Gew.%, jeweils bezogen auf das Gesamtgewicht der erfindungsgemäßen Sonnenschutz-Zusammensetzung.

Bevorzugt enthält die erfindungsgemäße Sonnenschutzzusammensetzung mehr als 0,01 Gew.-% eines oder mehrerer Sonnenschutzfilter, insbesondere UV-Filter.

Die Sonnenschutzfilter, insbesondere UV-Filter, können unter den organischen Filtern, den physikalischen Filtern und deren Gemischen ausgewählt werden.

Die erfindungsgemäße Sonnenschutz-Zusammensetzung kann insbesondere UV-A-, UV-B-Filter oder Breitbandfilter enthalten. Die eingesetzten UV-Filter können öllöslich oder wasserlöslich sein. Die folgende Liste der genannten UV-Filter ist selbstverständlich nicht limitierend.

Unter den UV-B-Filtern sind beispielsweise zu nennen
Salicylsäurederivate, besonders Homomenthylsalicylat, Octylsalicylat und Salicylsäure(4-isopropylbenzyl)ester;
Zimtsäurederivate, insbesondere 2-Ethylhexyl-p-methoxycinnamat, das von der Firma DSM unter der Bezeichnung Parsol MCX® erhältlich ist und 4-Methoxyzimtsäureisopentylester;
flüssige β,β'-Diphenylacrylatderivate, insbesondere 2-Ethylhexyl α,β'-diphenylacrylat oder Octocrylen, das von der Firma BASF unter der Bezeichnung UVINUL N539® erhältlich ist;
p-Aminobenzoesäurederivate, insbesondere 4-(dimethylamino)-benzoesäure (2-ethylhexyl)ester, 4-(dimethylamino)benzoesäureamylester;
3-Benzylidencampher-Derivate, insbesondere 3-(4-Methylbenzyliden)campher der von der Firma Merck unter der Bezeichnung EUSOLEX 6300® im Handel ist, 3-Benzylidencampher, Benzylidencampher sulfonsäure und Polyacrylamidomethyl Benzyli-dencampher;
2-Phenylbenzimidazol-5-sulfonsäure, die unter der Bezeichnung EUSOLEX 232® von Merck erhältlich ist;
1,3,5-Triazinderivate, insbesondere 2,4,6-Tris[p-(2'-ethylhexyl-1'-oxycarbonyl)anilinol-1,3,5-triazin, das von der Firma BASF unter der Bezeichnung UVINUL T150® angeboten wird, und Dioctylbutamidotriazon, das von der Firma Sigma 3V unter der Bezeichnung UVASORB HEB® angeboten wird;
Ester der Benzalmalonsäure, insbesondere 4-Methoxybenzalmalonsäuredi(2-ethylhexyl)ester und 3-(4-(2,2-bis ethoxycarbonylvinyl)-phenoxy)propenyl)-methoxysiloxan/ dimethylsiloxan-Copolymer, das von der Firma DSM unter der Bezeichnung Parsol® SLX erhältlich ist; und
die Gemische dieser Filter.

Als UV-A-Filter sind beispielsweise zu nennen:
Dibenzoylmethanderivate, besonders das 4-(t-Butyl)-4'-methoxydibenzoylmethan, das von der Firma DSM unter der Bezeichnung PARSOL 1789® angeboten wird und 1-Phenyl-3-(4'-isopropylphenyl)propan-1,3-dion;
Benzol-1,4-[di(3-methylidencampher-10-sulfonsäure)], gegebenenfalls ganz oder teilweise neutralisiert, unter der Bezeichnung MEXORYL SX® von Chimex im Handel;
2-(4'-Diethylamino-2'-hydroxybenzoyl)-benzoesäurehexylester (auch Aminobenzo-phenon);
Silanderivate oder Polyorganosiloxane mit Benzophenongruppen;
Anthranilate, besonders Menthylanthranilat, das von der Firma Symrise unter der Bezeichnung NEO HELIOPAN MA® angeboten wird;
Verbindungen, die pro Molekül mindestens zwei Benzoazolylgruppen oder mindestens eine Benzodiazolylgruppe enthalten, insbesondere 1,4-Bis-benzimidazolylphenylen-3,3',5,5'-tetrasulfonsäure sowie ihre Salze, die von der Firma Symrise im Handel sind;
Siliciumderivate von Benzimidazolylbenzazolen, die N-substituiert sind, oder von Benzofuranylbenzazolen, insbesondere: 2-[1-[3-[1,3,3,3-Tetramethyl-1-[(trimethylsilyl)oxy]disiloxanyl]-propyl] -1 H-benzimidazol-2-yl] -benzoxazol, 2-[1-[3-[1,3,3,3-Tetramethyl-1-[(trimethylsilyl)oxy]disiloxanyl]-propyl]-1H-benzimidazol-2-yl]-benzothiazol, 2-[1-(3-Trimethylsilanylpropyl)-1H-benzimidazol-2-yl]-benzoxazol, 6-Methoxy-1,1'-bis(3-trimethylsilanylpropyl)1H,1'H-[2,2']dibenzimidazolylbenzoxazol, 2-[1-(3-Trimethylsilanylpropyl)-1H-benzimidazol-2-yl]-benzothiazol, die in der Patentanmeldung EP-A-1 028 120 beschrieben sind;
Triazinderivate, insbesondere 2,4-bis-[5-1(dimethylpropyl)benzoxazol-2-yl-(4-phenyl)-imino]-6-(2-ethylhexyl)-imino-1,3,5-triazin, das von der Firma 3V unter der Bezeichnung Uvasorb®K2A angeboten wird; und
deren Gemische.

Als Breitbandfilter sind beispielweise zu nennen:
Benzophenonderivate, beispielsweise 2,4-Dihydroxybenzophenon (Benzophenon-1), 2,2',4,4'-Tetrahydroxybenzophenon (Benzophenon-2), 2-Hydroxy-4-methoxybenzophenon (Benzophenon-3), von der Firma BASF un-ter der Bezeichnung UNIVNUL M40® erhältlich, 2-Hydroxy-4-methoxybenzophenon-5-sulfonsäure (Benzophenon-4), sowie ihre Sulfonatform (Benzonphenon-5), von der Firma BASF unter der Bezeichnung UVINUL MS40® im Handel, 2,2'-Dihydroxy-4,4'-dimethoxybenzophenon (Benzophenon-6-), 5-Chlor-2-hydroxybenzophenon (Benzophenon-7-), 2,2'-Dihydroxy-4-methoxybenzophenon (Benzophenon-8), das Dinatriumsalz von 2,2'-Dihydroxy-4,4'-dimethoxybenzophenon-5,5'-disulfonsäure (Benzophenon-9-), 2-Hydroxy-4-methoxy-4'-methylbenzophenon (Benzophenon-10), Benzophenon-11, 2-Hydroxy-4-(octyloxy)-benzophenon (Benzophenon-12);
Triazinderivate, insbesondere das 2,4-Bis{[4-2-ethylhexyloxy)-2-hydroxy]-phenyl}-6-(4-methoxyphenyl)-1,3,5-triazin , das von der Firma BASF unter der Bezeichnung TINOSORB S® angeboten wird, das 2,2'-Methylen-bis[6-(2H-benzotriazol-2-yl)4-(1,1,3,3-tetramethylbutyl)phenol], das von der Firma BASF unter der Bezeichnung TINOSORB M® erhältlich ist; und das Phenyl-bis-diphenyltriazine und
2-(1H-benzotriazol-2-yl)-4-methyl-6-[2-methyl-3-[1,3,3,3-tetramethyl-1-[(trimethylsilyl)oxy]disiloxanyl]propyl]-phenol mit der INCI-Bezeichnung Drometrizole Trisiloxane.

Es können auch ein Gemisch von mehreren Filtern und ein Gemisch von UV-B-Filtern, UV-A-Filtern und Breitbandfilter sowie Gemische mit physikalischen Filtern verwendet werden.

Unter den physikalischen Filtern können beispielsweise die Sulfate des Bariums, Oxide von Titan, insbesondere Titandioxid, amorph oder kristallin in Form von Rutil und/oder Anatas, von Zink, von Eisen, von Zirkonium, von Cer, von Silicium, von Mangan oder deren Gemische angegeben werden.

Die Metalloxide können in Partikelform mit einer Größe im Mikrometerbereich oder Nanometerbereich (Nanopigmente) vorliegen. Die mittleren Partikelgrößen betragen für die Nanopigmente beispielsweise 5 bis 100 nm.

Die erfindungsgemäße Sonnenschutz-Zusammensetzungen können zusätzlich ein oder mehrere weitere Zusatzstoffe enthalten, die in der Kosmetik üblich sind, wie Antioxidantien, und/oder andere Hilfs- und Zusatzmittel wie beispielsweise Emulgatoren, grenzflächenaktive Stoffe, Entschäumer, Verdicker, Tenside, Wirkstoffe, Feuchthaltemittel, Füllstoff, Filmbildner, Lösemittel, Koaleszenzmittel, Aromastoffe, Geruchsabsorber, Parfüms, Gelbildner und/oder andere Polymerdispersionen wie beispielsweise Dispersionen auf Basis von Polyacrylaten, Füllstoffe, Weichmacher, Pigmente, Farbstoffe, Verlaufsmittel, Thixotropiemittel, Geschmeidigkeitsmittel, Weichmacher, Konservierungsmittel etc. Die Mengen der verschiedenen Zusatzstoffe sind dem Fachmann für den einzusetzenden Bereich bekannt und liegen beispielweise im Bereich von 0 bis 25 Gew. % bezogen auf das Gesamtgewicht der Sonnenschutz-Zusammensetzung.

Die erfindungsgemäße Sonnenschutz-Zusammensetzung kann auch sensorische Additiv enthalten. Unter sensorischen Additiven sind insbesondere farblose oder weiße, mineralische oder synthetische, lamellare, sphärische oder längliche inerte Partikel oder ein nicht-partikuläres sensorisches Additiv zu verstehen, welche z.B. die sensorischen Eigenschaften der Formulierungen weiter verbessern und beispielsweise ein samtiges oder seidiges Hautgefühl hinterlassen.

Die sensorischen Additive können in der erfindungsgemäßen Zusammensetzung beispielsweise in einer Menge von bis zu 10 Gew.-%, bevorzugt 0,1 bis 10 Gew.-% und noch bevorzugter 0,1 bis 7 %, jeweils bezogen auf das Gesamtgewicht der Zusammensetzung, enthalten sein.

Vorteilhafte partikuläre sensorische Additive im Sinne der vorliegenden Erfindung sind Talkum, Glimmer (Mica), Siliciumoxid, Kaolin, Stärke und deren Derivaten (beispielweise Tapiocastärke, Distärkephosphat, Aluminium- bzw. Natrium-Stärke Octenylsuccinat und dergleichen), pyrogene Kieselsäure, Pigmente, die weder hauptsächlich UV-Filter-noch färbende Wirkung haben, wie z.B. Bornitrid, Calciumcarbonat, Dicalciumphosphat, Magnesiumcarbonat, Magnesiumhydrogencarbonat, Hydroxyapatite, mikrokristalline Cellulose, Pulver von synthetischen Polymeren, wie Polyamide (beispielsweise die unter der Handelsbezeichnung "Nylon®" erhältlichen Polymere), Polyethylen, Poly-β-alanine, Polytetrafluorethylen ("Teflon®"), Polyacrylat, Polyurethan, Lauroyllysine, Silikonharz (beispielsweise die unter der Handelsbezeichnung "Tospearl®" der Firma Kobo Products Inc. erhältlichen Polymere), Hohlpartikel von Polyvinyliden/Acrylonitrile (Expancel® der Firma Akzo Nobel) oder Hohlpartikel von Siliciumoxid (Silica Beads® der Firma MAPRECOS).

Vorteilhafte nicht-partikuläre sensorische Additive können aus der Gruppe der Dimethiconole, z. B. Dow Corning 1503 Fluid der Fa. Dow Corning Ltd., der Siliconcopolymere, z. B. Divinyldimethico-ne/Dimethicone Copolymer, Dow Corning HMW 2220 der Fa. Dow Corning Ltd., oder der Si-liconelastomere, z. B. Dimethicone Crosspolymer, Dow Corning 9040 Silicone Elastomer Blend der Fa. Dow Corning Ltd., ausgewählt werden.

Die erfindungsgemäße Sonnenschutz-Zusammensetzung kann ein oder mehrere Feuchthaltemittel (Moisturizer) umfassen. Besonders vorteilhafte Feuchthaltemittel im Sinne der vorliegenden Erfindung sind beispielsweise Glycerin, Polyglycerin, Sorbit, Dimethylisosorbid, Milchsäure und/oder Lactate, insbesondere Natriumlactat, Butylenglykol, Propylenglykol, Biosaccaride Gum-1, Glycine Soja, Hydroxyethylharnstoff, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches unter der Bezeichnung Fucogel™ 1000 von der Gesellschaft SOLABIA S.A. erhältlich ist.

Besonders vorteilhaft im Sinne der vorliegenden Erfindung können Antioxidantien, wie zum Beispiel wasserlösliche Antioxidantien eingesetzt werden, wie beispielsweise Vitamine, z.B. Ascorbinsäure und deren Derivate. Ganz besonders vorteilhafte sind Vitamin E und dessen Derivate sowie Vitamin A und dessen Derivate.

Weitere vorteilhafte Wirkstoffe in der erfindungsgemäßen Zusammensetzung schließen ein α-Hydroxycarbonsäuren, wie Glykolsäure, Milchsäure, Äpfelsäure, Weinsäure, Citronensäure und Mandelsäure, β-Hydroxycarbonsäuren, wie Salicylsäure sowie deren acylierten Derivate, die 2-Hydroxyalkansäure und ihre Derivate; natürliche Wirkstoffe und/oder deren Derivate, wie z.B. alpha-Liponsäure, Folsäure, Phytoen, D-Biotin, Coenzym Q10, alpha-Glucosylrutin, Carnitin, Carnosin, natürliche und/oder synthetische Isoflavonoide, Kreatin, Kreatinin, Taurin und/oder [beta]-Alanin sowie 8-Hexadecen-1,16-dicarbonsäure, d.h. Dioic acid, CAS-Nummer 20701-68-2; vorläufige INCI-Bezeichnung Octadecendioic acid und/oder Licochalcon A und die Pflanzenextrakte.

Die vorliegende Erfindung wird an Hand von Beispielen erläutert, wobei sie nicht als einschränkend zu verstehen sind. Alle Mengenangaben, Anteile und Prozentanteile sind, soweit nicht anders angegeben, auf das Gewicht und die Gesamtmenge bzw. auf das Gesamtgewicht der Zusammensetzungen bezogen.

### Beispiele:

### Eingesetzte Materialien:

PUD 1 (Vergleich): Polyurethan-Dispersion (Feststoffgehalt 40 Gew.-%) basierend auf synthetisch hergestelltem Polyesterpolyol 1, Dicyclohexylmethan-Diisocyanat, Ethylendiamin und Aminoethansulfonsäure-Natriumsalz, Polyurethan ist zu 100 mol-% synthetisch.

PUD 2 (erfindungsgemäß): Polyurethan-Dispersion (Feststoffgehalt 30 Gew.-%) basierend auf biobasiertem Polyesterpolyol 2, Isophoron-Diisocyanat, Isophorone-Diamine und und Aminoethansulfonsäure-Natriumsalz, Polyurethan ist zu 55 mol-% bio-basiert.

PUD 3 (erfindungsgemäß): Polyurethan-Dispersion (Feststoffgehalt 40 Gew.-%) basierend auf biobasiertem Polyesterpolyol 2, Dicyclohexylmethan-Diisocyanat, Ethylendiamin und Aminoethansulfonsäure-Natriumsalz, Polyurethan ist zu 60 mol-% bio-basiert.

Polyesterpolyol 1 (Vergleich): synthetisch hergestellt aus Adipinsäure, Neopentylglykol und 1,6-Hexandiol
Polyesterpolyol 2: (erfindungsgemäß): biobasierter Polyesterpolyol aus Bernsteinsäure, 1,4-Butandiol und Neopentylglykol

### DSC-Messungen:

Es wurden die Glasübergangstemperaturen Tg des Vergleichs-Polyesterpolyols 1 und des erfindungsgemäßen Polyesterpolyols 2, sowie der Vergleichs-Polyurethan-Dispersionen PUD 1 und der erfindungsgemäßen Polyurethandispersionen PUD 2 und PUD 3, jeweils als getrockneter Film, nach folgender Methode gemessen:
Die Bestimmung von physikalischen Umwandlungen wie Schmelzpunkten oder Glasübergangstemperaturen T_{g} erfolgt über Messung der Wärmekapazität in Abhängigkeit von der Temperatur mit einem Kalorimeter DSC3+e der Firma Mettler-Toledo. Die Temperatur und die Schmelzenthalpie werden über n-Heptan, Indium, Blei und Zink kalibriert. Als Spülgas wird Stickstoff mit einer Flussrate von 20 ml/min verwendet. Die Kühlung erfolgt über flüssigem Stickstoff. Der Temperaturgradient beträgt 20K/min. Gemessen wird im Temperaturbereich zwischen -70 und +150 °C (2 Aufheizungen).

Die Messungen erfolgen mit der Anlieferform, d.h. direkt mit den wässrigen Polyurethan-Dispersionen, ohne Vorkonditionierung. Die Probeneinwaagen betragen zwischen 10 und 12 mg Probenmasse in einem hermetisch verschließbaren, gelochten Aluminiumstandardtiegel.

Auswertung: die Glasübergangstemperatur T_{g} wurde als die Temperatur bei halber Höhe des Glasübergangs bei der zweiten Aufheizung bestimmt.

Die Ergebnisse der Messungen sind in Tabelle 1 dargestellt.

**Tabelle 1: DSC-Ergebnisse**

| **Nr.** | **Bezeichnung** | **T_{g} [°C]** |
|---|---|---|
| **V1** | Polyesterpolyol 1 | -60,1 |
| **2** | Polyesterpolyol 2 | -38,6 |
| **V3** | PUD 1 | -44,1 |
| **4** | PUD 2 | -16,5 |
| **5** | PUD 3 | -21,0 |

| | | |
|---|---|---|
| V Vergleich | | |

### Lichtschutzfaktor-Werte (SPF-Werte):

Die SPF-Werte einer Öl-in-Wasser Emulsion enthaltend die jeweilige PUD 1, 2 oder 3 wurden in Vivo gemäß ISO 24444:2010 auf 5 Probanden bestimmt.

Die einzelnen Öl-in-Wasser Emulsion dazu wurden wie folgt hergestellt: Alle Bestandteile der Phase A (siehe Tabelle 2) wurden unter Rühren in einem Becher zugegeben und auf 80 °C erhitzt wurden. In einem separaten Gefäß wurde Phase B (siehe Tabelle 2) auf 90 °C erhitzt. Phase B wurde unter kontinuierlichem Mischen in Phase A überführt. Phase C (siehe Tabelle 2) wurde zugegeben, die Mischung zwei Minuten lang homogenisiert und dann unter kontinuierlichem Mischen bei 300 U/min auf Raumtemperatur abgekühlt. Der pH-Wert wurde überprüft und mit NaOH (10 gew.-%ige, wässrige Lösung) auf 6,5 bis 7,0 eingestellt. Die Bestandteile der Phase D (siehe Tabelle 2) werden nacheinander zugegeben. Die Emulsion wurde weitere 5 Minuten gerührt.

**Tabelle 2: Komponenten der Öl-in-Wasser Emulsionen**

| **Phase** | **Komponente (Bezeichnung nach INCI)** | **Menge [Gew.-%]** |
|---|---|---|
| **A** | Wasser | 35,40 |
| | Tetranatrium-Glutamat-Diacetat | 0,50 |
| | Phenylpropanol, Propandiol, Caprylylglycol, Tocopherol | 1,00 |
| | Glycerin | 3,00 |
| | Zitronensäure | 0,10 |
| | Propylenglycol | 5,00 |
| | Natriumstearinglutamat | 1,00 |
| **B** | C12-15-Alkyl-benzoesäureester | 7,00 |
| | Diethylamino-hydroxybenzoyl-hexyl-benzoesäureester | 6,00 |
| | Ethylhexyl-salicylsäureester | 5,00 |
| | Glyceryl-stearinsäureester | 2,00 |
| | Dibutyl-adipinsäureester | 7,00 |
| | Homosalate | 7,00 |
| | Ethylhexyl-triazon | 5,00 |
| **c** | Simmondsia Chinensis (Jojoba)-Öl | 3,00 |
| | Xanthan | 0,30 |
| **D** | Panthenol | 0,50 |
| | Tocopheryl-essigsäureester | 0,20 |
| | Polyurethan (als Feststoff) | 2,40 |
| | Alkohol, denaturiert | 5,00 |
| **Summe** | | 100,00 |

Die Ergebnisse der Messung sind in Tabelle 3 dargestellt. PUD 2 und PUD 3 zeigen jeweils einen SPF-Boosting Effekt, der auf dem gleichen Niveau wie der von PUD 1 ist. Der in-vivo SPF-boosting-Wert für die Öl-in-Wasser Emulsionen mit PUD 2 oder PUD 3 ist jeweils signifikant höher als der in-vivo SPF-Wert für die gleiche Emulsion ohne Polyurethan (blank).

**Tabelle 3: Resultate der in-vivo Messungen**

| **Nr.** | **Öl-in-Wasser Emulsionen** | **in-vivo SPF Wert** |
|---|---|---|
| **V6** | ohne Polyurethan (blank) | 17,7 |
| **V7** | mit PUD 1 | 41,1 |
| **8** | mit PUD 2 | 32,8 |
| **9** | mit PUD 3 | 39,2 |

| | | |
|---|---|---|
| V Vergleich | | |

### Wasserfestigkeit:

Die Wasserfestigkeit der Test Öl-in-Wasser Emulsionen aus dem vorhergehenden Beispiel wurde nach der Colipa Methode aus Dezember 2005 bestimmt.

Im Gegensatz zu der Öl-in-Wasser-Emulsion ohne Polyurethan (blank) ist die Emulsion enthaltend PUD 3 wasserfest, d.h. nach 2x 20 min im Wasserbad sind noch mehr als 50% des Ausgangs-SPF-Wertes enthalten.

## Patentansprüche

1. Sonnenschutz-Zusammensetzung, enthaltend mindestens ein Polyurethan, erhältlich durch Umsetzung eines oder mehrerer wasserunlöslicher, nicht wasserdispergierbarer, isocyanatfunktioneller Polyurethanprepolymere A mit einer oder mehreren aminofunktionellen Verbindungen B), **dadurch gekennzeichnet, dass** das Polyurethanprepolymere A) erhältlich ist durch Umsetzung von einem oder mehreren Polyesterpolyolen mit einer Glasübergangstemperatur T_{G} von mindestens -50 °C und einem oder mehreren Polyisocyanaten.

2. Sonnenschutz-Zusammensetzung nach Anspruch 1, **dadurch gekennzeichnet, dass** ein oder mehrere isocyanatfunktionelle Polyurethanprepolymere A) im Wesentlichen weder ionische noch ionogene Gruppen aufweisen.

3. Sonnenschutz-Zusammensetzung nach Anspruch 1 oder 2, **dadurch gekennzeichnet, dass** die aminofunktionellen Verbindungen B) aus primären und/oder sekundären Aminen und/oder Diaminen ausgewählt werden.

4. Sonnenschutz-Zusammensetzung nach einem der Ansprüche 1 bis 3, **dadurch gekennzeichnet, dass** die aminofunktionelle Verbindungen B) mindestens ein Diamin umfassen.

5. Sonnenschutz-Zusammensetzung nach einem der Ansprüche 1 bis 4, **dadurch gekennzeichnet, dass** die aminofunktionellen Verbindungen B) ausgewählt werden aus aminofunktionellen Verbindungen B2), die ionische und/oder ionogene Gruppen aufweisen, und aminofunktionellen Verbindungen B1), die keine ionische und/oder ionogene Gruppen aufweisen.

6. Sonnenschutz-Zusammensetzung nach einem der Ansprüche 1 bis 5, **dadurch gekennzeichnet, dass** die aminofunktionellen Verbindungen B) wenigstens eine aminofunktionelle Verbindung B2) umfassen, die ionische und/oder ionogene Gruppen aufweist, bevorzugt 2-(2-Aminoethylamino)ethansulfonsäure und/oder deren Salze.

7. Sonnenschutz-Zusammensetzung nach einem der Ansprüche 1 bis 6, **dadurch gekennzeichnet, dass** die aminofunktionellen Verbindungen B) wenigstens eine aminofunktionelle Verbindung B1) umfassen, die keine ionische und/oder ionogene Gruppen aufweisen, bevorzugt ein Diamin, dass keine ionischen und/oder ionogenen Gruppen aufweist.

8. Sonnenschutz- Zusammensetzung nach einem der Ansprüche 1 bis 7, **dadurch gekennzeichnet, dass** die aminofunktionellen Verbindungen B) sowohl aminofunktionelle Verbindungen B2), die ionische und/oder ionogene Gruppen aufweisen, als auch aminofunktionellen Verbindungen B1), die keine ionischen und/oder ionogenen Gruppe aufweisen, umfassen.

9. Sonnenschutz-Zusammensetzung nach einem der Ansprüche 1 bis 8, **dadurch gekennzeichnet, dass** das Polyurethan mindestens eine Sulfonsäure und/oder Sulfonatgruppe, bevorzugt eine Natriumsulfonatgruppe enthält.

10. Sonnenschutz-Zusammensetzung nach einem der Ansprüche 1 bis 9, **dadurch gekennzeichnet, dass** sie eine oder mehrere Sonnenfilter-Substanzen enthält.

11. Verwendung einer Zusammensetzung, enthaltend mindestens ein Polyurethan, erhältlich durch Umsetzung eines oder mehrerer wasserunlöslicher, nicht wasserdispergierbarer, isocyanatfunktioneller Polyurethanprepolymere A) mit einer oder mehreren aminofunktionellen Verbindungen B), wobei das Polyurethanprepolymere A) erhältlich ist durch Umsetzung von einem oder mehreren Polyesterpolyolen mit einer Glasübergangstemperatur T_{G} von mindestens -50 °C und einem oder mehreren Polyisocyanaten, als Sonnenschutz-Zusammensetzung.

12. Polyurethan, erhältlich durch Umsetzung eines oder mehrerer wasserunlöslicher, nicht wasserdispergierbarer, isocyanatfunktioneller Polyurethanprepolymere A mit einer oder mehreren aminofunktionellen Verbindungen B), **dadurch gekennzeichnet, dass** das Polyurethanprepolymere A) erhältlich ist durch Umsetzung von einem oder mehreren Polyesterpolyolen mit einer Glasübergangstemperatur T_{G} von mindestens -50 °C und einem oder mehreren Polyisocyanaten.

13. Kosmetisches Verfahren zum Schutz der Haut vor negativen Wirkungen der Sonnenstrahlung, welches das Auftragen einer Zusammensetzung, enthaltend mindestens ein Polyurethan, erhältlich durch Umsetzung eines oder mehrerer wasserunlöslicher, nicht wasserdispergierbarer, isocyanatfunktioneller Polyurethanprepolymere A) mit einer oder mehreren aminofunktionellen Verbindungen B), wobei das Polyurethanprepolymere A) erhältlich ist durch Umsetzung von einem oder mehreren Polyesterpolyolen mit einer Glasübergangstemperatur T_{G} von mindestens -50 °C und einem oder mehreren Polyisocyanaten, auf die Haut umfasst.

14. Kosmetisches Verfahren nach Anspruch 13, **dadurch gekennzeichnet, dass** die Zusammensetzung nach dem Auftrag auf die Haut zumindest teilweise auf dieser verbleibt.
